# EUROPEAN PATENT APPLICATION

(11) **EP 2 570 405 A1**
(43) Date of publication of application: **20.03.2013**
(21) Application number: 11181280.6
(22) Date of filing: 14.09.2011
(51) Int. Cl.: C07D 239/47, C07D 239/69, C07D 405/12, C07D 487/04, A61K 31/519

(54) **Synthesis of Triazolopyrimidine Compounds**

(71) Applicant: LEK Pharmaceuticals d.d., 1526 Ljubljana (SI)
(72) Inventor: The designation of the inventor has not yet been filed
(74) Representative: Kunic Tesovic, Barbara

(57) **Abstract**

The present invention relates to the filed of organic synthesis and describes the synthesis of specific triazolopyrimidine compounds and intermediates thereof as well as related derivatives, suitable for the preparation of ticagrelor (TGC)

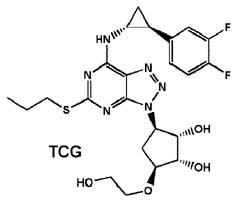

## Description

The present invention relates to the filed of organic synthesis, in particular to the synthesis of specific triazolopyrimidine compounds and intermediates thereof as well as related derivatives.

An important triazolopyrimidine compound is ticagrelor (TCG; Brilinta^{®}; 3-[7-[[(1*R*,2*S*)- 2-(3,4-difluorophenyl)cyclopropyl]amino]-5-(propylthio)-3*H-1*,2,3-triazolo[4,5-d]pyrimidin-3-yl]-5-(2-hydroxyethoxy)-(1*S*,2*S*,3*R*,5*S*)-1,2-cyclopentanediol) having the following structural formula.

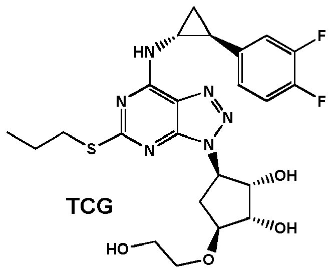

Ticagrelor shows pharmaceutical activity by functioning as a P2Y12 receptor antagonist and thus is indicated for the treatment or prevention of thrombotic events, for example stroke, heart attack, acute coronary syndrome or myocardial infection with ST elevation, other coronary artery diseases and arterial thrombosis as well as other disorders related to platelet aggregation (WO 00/34283).

The synthesis of ticagrelor (TCG) is demanding. There are five to six known synthetic variants, which are described in the basic patent application WO 00/34283, an improved one in patent application WO 01/92263, and a further improved one in patent application WO 10/030224 respectively derived from the originator AstraZeneca, while two are published in a "deutero" patent application WO 11/017108 of Auspex Pharmaceuticals. Further, there is one synthetic path published in a scientific journal (Bioorg. Med. Chem. Lett. 2007, 17, 6013-6018).

The first synthesis of TCG as described in WO 00/34283 is depicted in scheme 1 below.

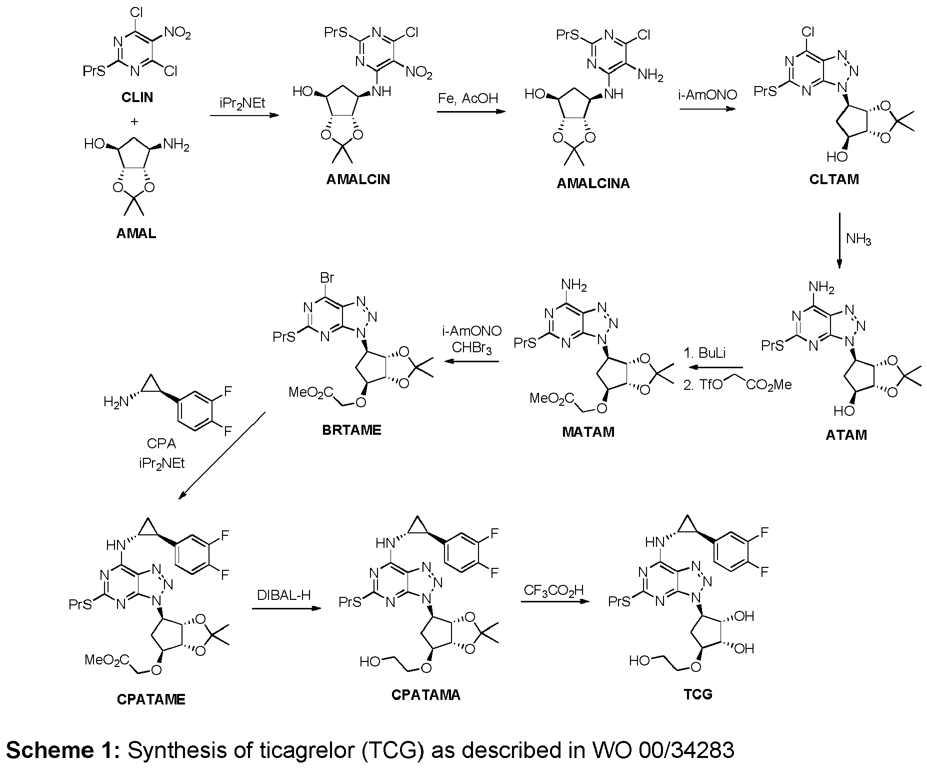

This nine step synthesis of ticagrelor (**TCG**) as described in WO 00/34283 (Scheme 1) starts with a reaction between **CLIN** and **AMAL.** In the presence of diizopropylethylamine (*i*Pr₂Net) **AMALCIN** is formed, which is in then reduced with iron (Fe) in acetic acid to **AMALCINA.** In the next step **CLTAM** is formed using isopentyl nitrite (*i*AmONO). Next, **ATAM** was prepared using ammonia, and side chain was introduced (**MATAM**) using *n*-butyllithium and methyl 2-(((trifluoromethyl)sulfonyl)oxy)acetate, which was previously prepared by reaction between methyl glycolate and triflic anhydride. In next step **BRTAME** is formed using *i*AmONO and CHBr₃, followed by the aromatic nucleophilic substitution of Br with **CPA** in the presence of *i*Pr₂NEt to form **CPATAME.** This is then reduced to **CPATAMA** using DIBAL-H. Deprotection of diol group in the presence of trifluoroacetic acid in the final step leads to **TCG.**

This synthetic path is very long (9 steps, not including reagents preparation) and uses toxic compounds like CHBr₃, triflic anhydride, and methyl 2-(((trifluoromethyl)sulfonyl)oxy)acetate. The introduction of the methoxycarbonylmethyl group (reaction from **ATAM** to **MATAM**) is very difficult due to poor chemoselectivity, as the amino group also reacts with 2-(((trifluoromethyl)sulfonyl)oxy)acetate.

An improved synthesis of ticagrelor (**TCG**) is described in WO 01/92263 (see Scheme 2). In this process the hydroxyethyl side chain is introduced at the beginning of the synthesis by a three step reaction path from **AMAL** to **AMALA,** which is then reacted with **CLINA** (prepared from **CLIDA)** in presence of triethylamine (Et₃N) to form **AMALCINAA.** The triazole ring of **CLTAM** is formed with NaNO₂ in acetic acid, and then Cl is exchanged with **CPA** to form **CPATAMA.** In the final step **TCG** is prepared via deprotection using HCl.

This improved process still has substantial length (7-8 steps). In **AMALA** synthesis the benzyloxycarbonyl protection (*Cbz*) is used, which is then removed in the third step using hydrogenation with Pd/C as a catalyst. Hydrogenation with Pt/C as a catalyst is also used in the reduction of **CLIDA** to **CLINA.**

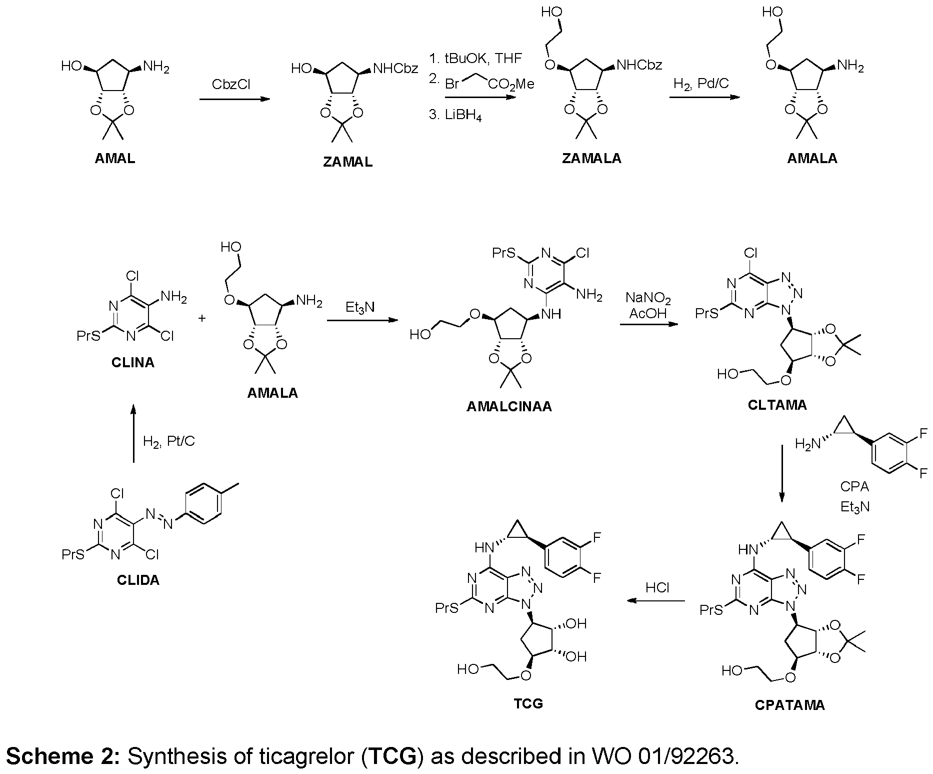

Another improved synthetic path is described in WO 10/030224 (Scheme 3). The key steps in this process are reduction of **CLIN** to **CLINA** or **AMALCINO** to **AMALCINAA** using hydrogen gas and platinum vanadium catalyst. The introduction of the hydroxyethyl side chain to **AMAL** to form **AMALA,** cyclization, substitution of Cl atom of **CLTAMA** with **CPA** and final acidic deprotection are the same as in WO 01/92263.

This further improved process to **TCG** has 8 reaction steps. Like in WO 01/92263, there are used the *Cbz* protecting group and heavy metals as catalysts like Pd, Pt and/or V.

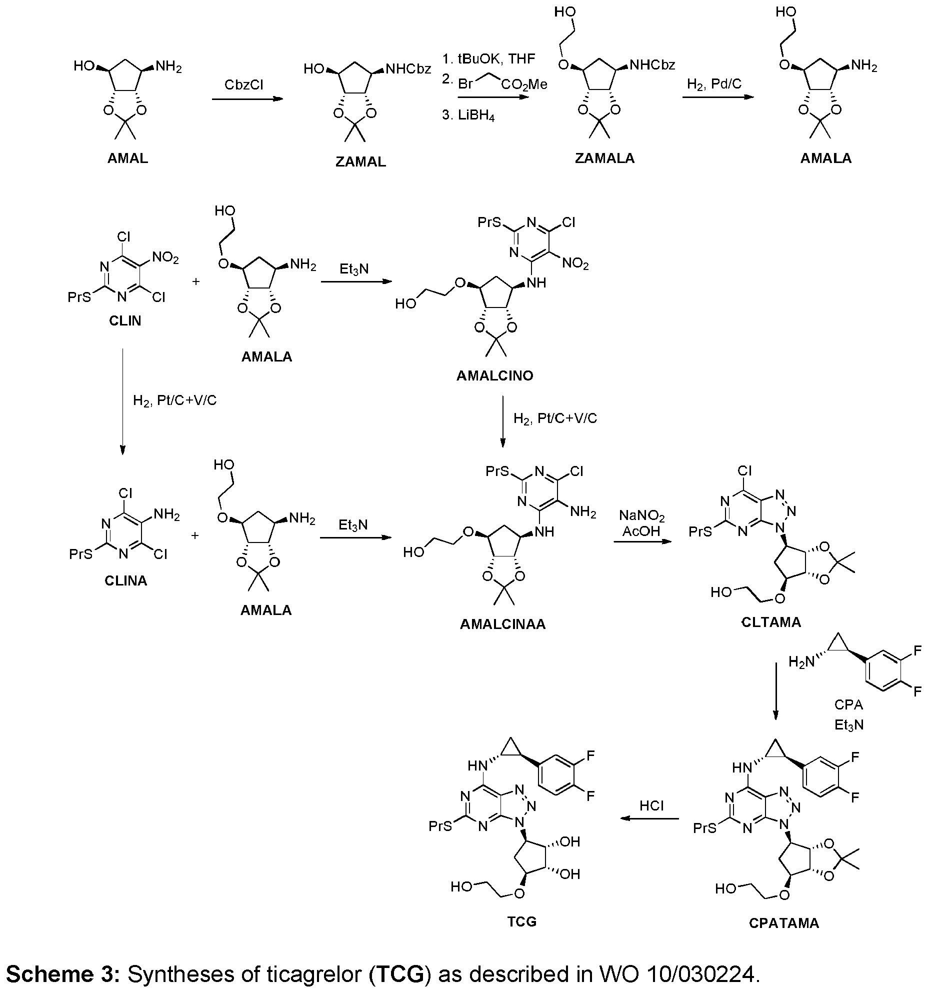

AstraZeneca published a synthetic path (Scheme 4) to ticagrelor (**TCG**) in Bioorg. Med. Chem. Lett. 2007, 17, 6013-6018. Intermediates in this process are similar to those described in WO 01/92263. There is difference in formation of triazolo ring of **CLTAMA** where *i*AmONO is used, and difference in deprotection in the last step.

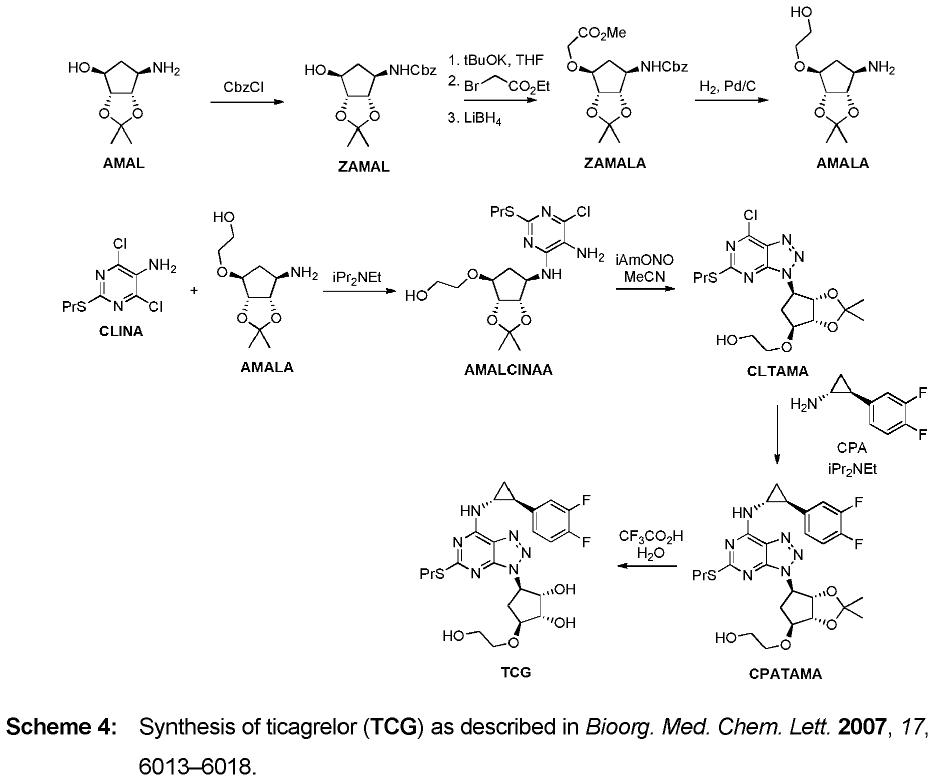

Another synthetic variant (Scheme 5) to ticagrelor (**TCG**) is described in WO 11/017108 by Auspex Pharmaceuticals. In nine step synthesis they prepared AMAL**E** through deprotection of **ZAMALE** using hydrogen gas and Pd/C, which was then reduced to **AMALA** with LiAlH₄. **AMALCINO** was prepared without presence of base, further steps are similar to those published in WO 01/92263.

Still another synthetic variant (Scheme 6) to obtain ticagrelor with deuterated hydroxyethyl group (**TCGD**) is also described in WO 11/017108 by Auspex Pharmaceuticals.

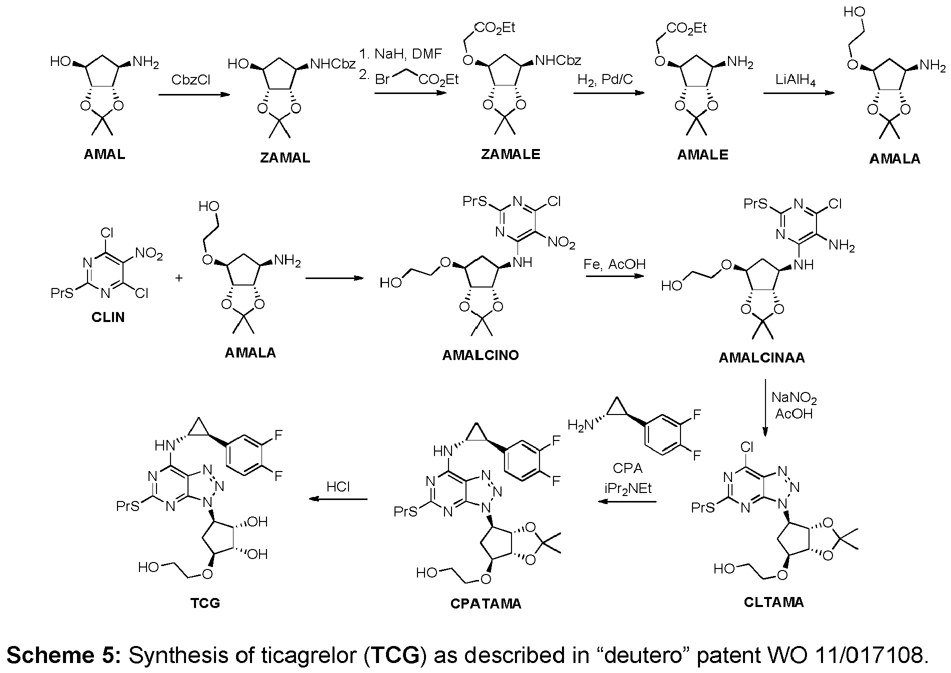

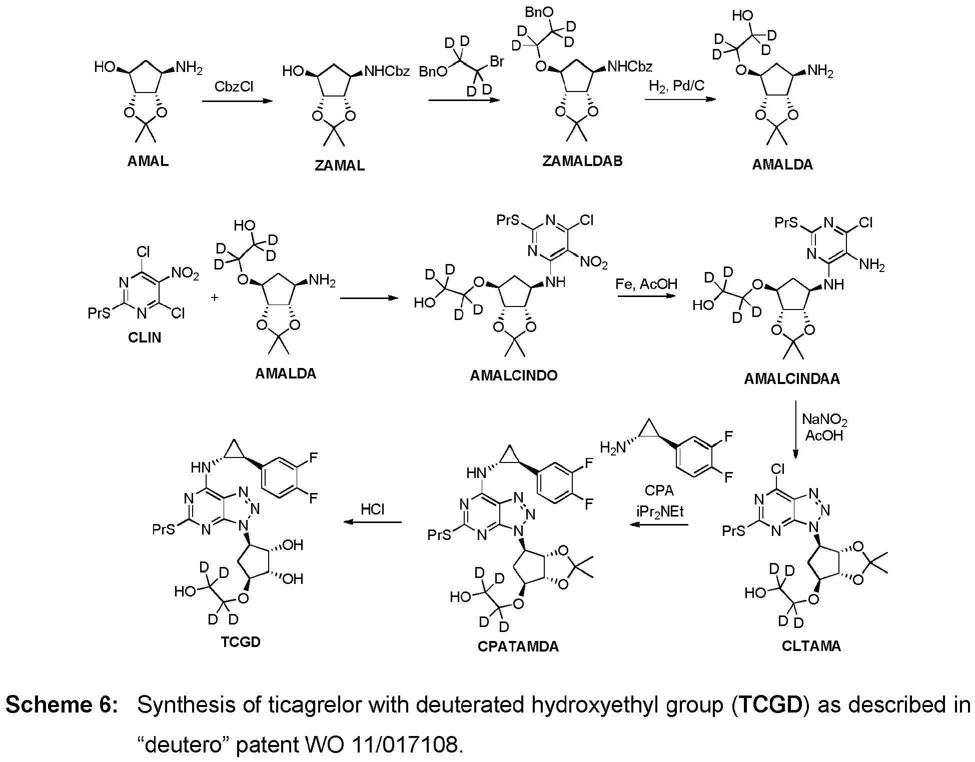

As becomes apparent from the above, a major drawback of the hitherto known synthesis schemes for the preparation of ticagrelor is that the synthesis is long.

### Summary of the Invention

The object of the present invention was to provide an industrially applicable and economically improved process for obtaining ticagrelor.

The present invention provides a process for the preparation of a compound of formula Va. 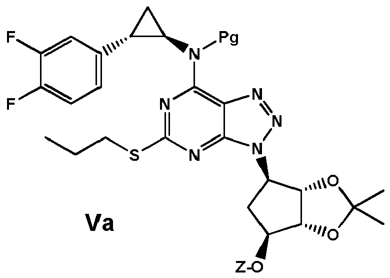 wherein Pg is an amino protecting group, and Z is hydrogen, hydroxyethyl or a group convertible to hydroxyethyl, the process comprising the steps of:
(i) reacting a compound of formula II 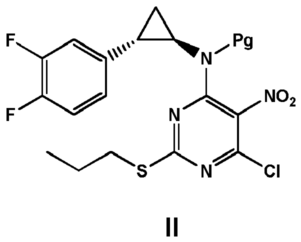 wherein Pg is defined as above, with a compound of the formula VIIa 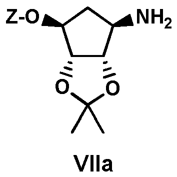 wherein Z is defined as above, to obtain a compound of formula IIIa 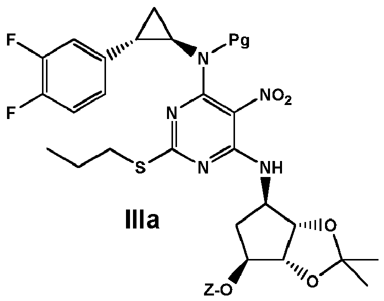 wherein Pg and Z are as defined above,
(ii) reducing the nitro group in the compound of formula IIIa to an amino group to obtain a compound of formula IVa, and 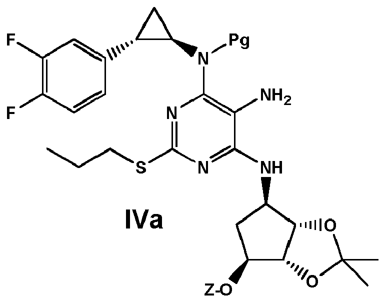
(iii) nitrosation of the compound of formula IVa to obtain the compound of formula Va.

The process defined above allows for preparation or synthesis of ticagrelor with an industrially applicable and economically improved process. Preferred embodiments will be described below. The present invention further provides novel compounds that are highly useful as key intermediates in the preparation or synthesis of ticagrelor.

### Description of the Invention and of Preferred Embodiments

Aspects, advantageous features and preferred embodiments of the present invention will be described in further detail below, noting however that such aspects, advantages features as well as embodiments and examples are presented for illustrative purposes only and shall not limit the invention in any way.

The introduction of a protective group on the amino group of the cyclopropane ring is a significant point of the present invention, which is a novel feature common to the key steps of the synthetic preparation as well as to the intermediate compounds mentioned above. This crucial point significantly distinguishes over every prior art synthesis in which a substitution reaction of the pyrimidine ring with cyclopentane is carried out first, and only then the triazole ring is created with a final substitution with the cyclopropane ring. This sequence of the reaction steps in the prior art solutions needs to be followed exactly, otherwise a selectivity of the triazole ring formation is eliminated, or wrong triazole systems are obtained. Introduction of the protective group on the amino group of cyclopropane ring according to the present invention however enables selectivity of the triazole ring formation regardless of the step conducted after the protective group is introduced. The synthesis and the special types of intermediate compounds are already suitably protected for the selective introduction of the cyclopentane ring and/or the selective introduction of the hydroxylethyl group.

In particular, the process according to the present invention reduces the number of the required reaction steps. It is possible to proceed in a short 3-4 step process, contrary to prior art processes requiring 7 steps or more. Further, while prior art syntheses use toxic or expensive reagents, and most of them require the use of hydrogen gas and heavy metals, it is possible according to the present invention to avoid the use of hydrogen gas, heavy metals and expensive reagents. Such avoidance of using hydrogen gas and/or heavy metals during the whole synthesis of ticagrelor, or in the preparation of precursor compounds disclosed herein, thus constitutes a preferred embodiment of the present invention.

A further significant advantage of the present invention resides in the possibility that several steps can be performed through one-pot conversions, without the need of isolation or separation of intermediate compounds, which one-pot system therefore constitutes a preferred embodiment of the present invention.

Accordingly, the possibility of reducing the number of required reaction steps, of increasing reaction selectivity, and of simplifying reactions respectively strongly contributes to provide an improved industrially applicable and economically beneficial process for obtaining triazolopyrimidine compounds and specifically ticagrelor.

According to a preferred embodiment, the compound of formula II is prepared by comprising the steps of
(0-1) providing a compound of formula I 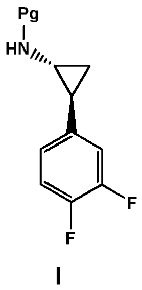 wherein Pg is an amino protecting group, and
(0-2) reacting the compound of formula I with a compound of the formula 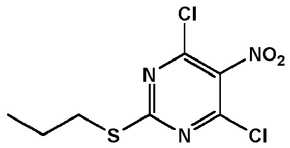 to obtain the compound of formula II.

It has been found that various amino-protecting groups can be efficiently introduced at this early stage of the synthesis, wherein such amino protecting groups are particularly suited and consistent with the subsequent reaction steps, and allowing to increase reaction selectivity and making reaction simplifications through one-pot conversions possible.

Alternatively, the compound of formula II can be obtained by first carrying out a substitutional reaction with the pyrimidine ring and subsequently introducing the protecting group, by comprising the steps of:
(0-1') providing a compound of formula Ia 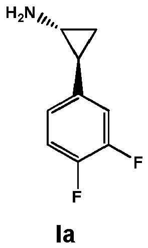
(0-2') reacting the compound of formula Ia with a compound of the formula 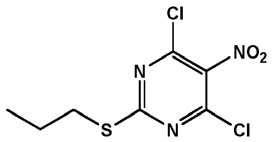 to obtain a compound of formula IIa, 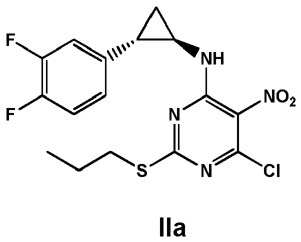 and
(0-3') introducing an amino protecting group Pg to obtain the compound of formula II.

A summary of the afore-mentioned ways to prepare the compound of formula II is shown in the following scheme 7 below.

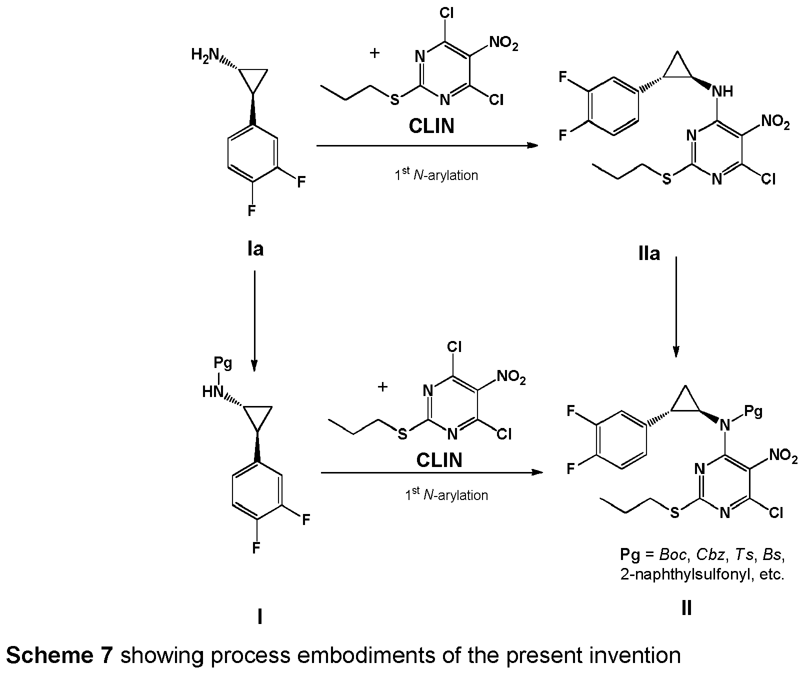

As set forth above, it is possible and corresponds to a particularly preferred embodiment of the present invention, that steps (i) to (iii) specified above, and optionally also the preparation of the compound of formula II, are carried out in one pot. Thus, while of course separation or isolation of any of the intermediate compounds of formulae IIIa and IVa and optionally of the compound of formula II can be carried out to obtain such compounds as useful intermediate compounds, this can be beneficially dispensed with if desired. This preferred embodiment is not only economically beneficial by the feature that one-pot synthesis is made possible; it is especially advantageous due to the generally amorphous nature of the intermediate compounds, which would make the purification difficult using non-chromatographic means, while the use of chromatographic means would again render the whole process less economically acceptable. Furthermore, the protecting group Pg may be selected in such way that the intermediate of the critical isolable step is solid and recrystallizable, hence no need for using chromatography as a purification method exists anymore.

A further preferred embodiment, which is associated with additional advantages, is based on the beneficial possibility to carry out steps (i) and (ii), optionally also steps (0-1) to (0-2) or (0-1') to (0-3') generally under basic conditions in the presence of bases, which renders the synthetic steps consistent and further facilitates one-pot methodologies. More specifically, all chemical steps from the start shown above up to the compound of formula IV are most efficient in the presence of bases of various strengths, more preferably in subsequent steps consistent with, or allowing, decreasing basicity. For example, a proper base, which can be used for the deprotonation of the compound of formula Ia is sodium hydride, while a suitable base which can be used as a hydrogen chloride scavenger in reaction step (i) (i.e. during the N-arylation of the compound of formula VIIa) can be selected from tertiary amines, alkali carbonates, or alkali phosphates, or from other poorly nucleophilic bases. Subsequently, mildly basic conditions, preferably using alkali carbonates, are well suited also for the nitro group reduction in step (ii), using for example sulphur-based reducing agents such as sodium dithionite or formamidine sulfinic acid (thiourea dioxide). Subsequently, and again consistent with a preferred one-pot methodology, the nitrosation step (iii) can be performed by shifting to mildly acidic conditions, suitably achieved for example by the addition of acetic acid, using appropriate nitrosating agents such as sodium nitrite, or it can be carried out as a alternative step by heating the solution of the crude product of the compound of formula IV in the presence of an alkyl nitrite such as the readily available reagent isopentyl nitrite.

Further advantageous embodiments of the process according to the present invention are based on the synthetic possibility that useful synthetic options are allowed, depending on which cyclopentane substituent "Z" is used, and depending on what substituent "Z" is used at which stage of the synthesis. The possible synthetic options become more apparent by the illustrations of possible synthetic embodiments shown in the following scheme 8:

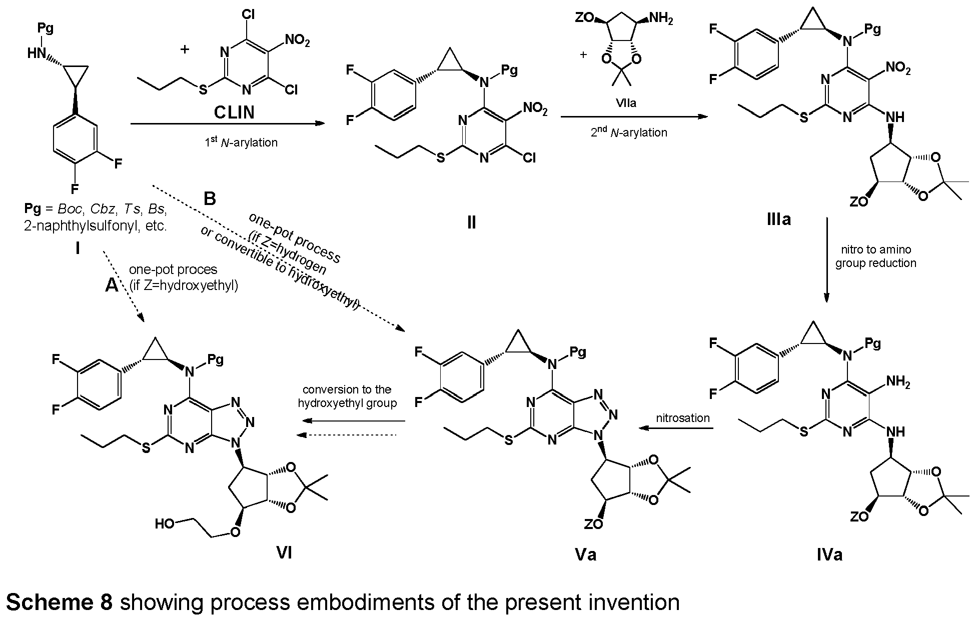

More specifically, if the compound of formula VIIa, that is used in the reaction step (i) to be reacted with the compound of formula II, already has a hydroxyethyl or a group convertible to hydroxyethyl as the group "Z", a one-pot process made feasible according to a preferred embodiment of the present invention already yields a desirable precursor compound of ticagrelor (see dashed arrow line A shown in reaction scheme 8), which precursor compound then only needs to be subjected to deprotection reactions for removing Pg and the vicinal hydroxyl protecting group at the cyclopentane ring, respectively. If, alternatively, "Z" in the compound of formula VIIa is hydrogen, then the molecular assembly sequence allows for a facilitated introduction of the hydroxyethyl group when it is done at a later stage of the synthesis, which is made possible because the described advantageous and preferred synthetic embodiments are still consistent with a one-pot process (see dashed arrow line B, and the subsequent conversion of the Z group in the compound of formula Va to the hydroxyethyl group as shown in scheme 8). Therefore, in the afore-mentioned alternative and economical routes to precursors of ticagrelor or ticagrelor itself, such preferred embodiments of the present invention constitute particularly suitable solutions for the synthetic challenge arising when the hydroxyethyl side chain is introduced at an early or at a later stage of the synthetic route.

Therefore, the advantageous possibility to join numerous steps into one-pot synthetic schemes is a surprising and unexpected effect of the present invention that results just from introducing the protective group to the amine moiety bound to the cyclopropane ring (denoted by the "Pg" protecting groups in the synthetic schemes), which protecting group "Pg" is present throughout the relevant intermediate compounds of formulas Ia-VIa (or as later shown in formulas I-VI).

According to another embodiment, alternatively, a compound of formula Vllb can be added to the compound of formula II to yield a compound of formula IIIb (Scheme 9). Vllb already has a hydroxyethyl group, while the hydroxyl groups at the cyclopentane ring are not protected.

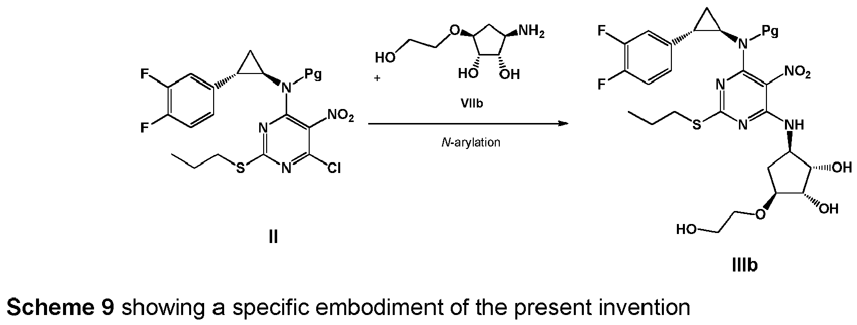

According to the present invention, the group "Pg" can be selected from the group of the group of oxycarbonyl-type amino protecting groups and sulfonyl-type amino protecting groups, without being limited thereto. According to further preferred embodiments, Pg can be selected from the group consisting of tert-butyloxycarbonyl (Boc), carbobenzyloxy (Cbz), benzenesulfonyl (Bs), p-toluenesulfonyl (Ts), and 2-naphthylsulfonyl.

For preparing a ticagrelor (TCG) with the formula VIII shown below, the compound of formula V or VI as described above is subjected to the deprotection reaction(s) in order to remove "Pg" and the vicinal hydroxyl protecting group at the cyclopentane ring, respectively. The deprotection reaction(s) can proceed at the same time, to concurrently remove both "Pg" and the vicinal hydroxyl protecting group at the cyclopentane ring, for example using acids such as HCl or phosphoric acid in a suitable organic solvent, for example alcohols such as methanol or ethanol. If desired, a salt, a cocrystal or a complex of the compound of formula VIII (ticagrelor, TCG) can be optionally formed.

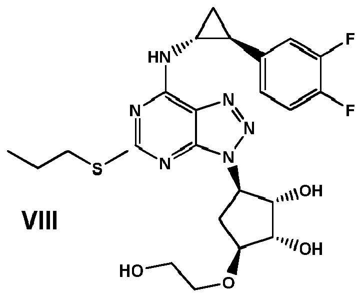

The ticagrelor compound prepared according to the invention may be used or administered on its own, preferably it is administered as a pharmaceutical composition comprising ticagrelor and a pharmaceutically acceptable excipient and/or carrier. Further, the ticagrelor compound prepared according to the invention may be combined with other drugs, especially drugs having activity against platelet aggregation or thrombolytic events.

In a further aspect of the present invention, a pharmaceutical composition comprising the compound of formula VIII (ticagrelor, TCG) or a salt thereof is prepared by comprising the steps of preparing the compound of formula VIII or a salt thereof as described above, and mixing the compound of formula VIII or a salt thereof with a pharmaceutically acceptable carrier and/or excipient. The administration form can be suitably chosen, e.g. a form suitable for oral, parenteral, rectal administration and/or administration by inhalation, and the dosage form may be solid, liquid, or powdery. Therefore, the pharmaceutical composition comprising ticagrelor compound prepared according to the invention may suitably be in the form of tablets, pills, capsules, syrups, powders or granules for oral administration; or as sterile parenteral or subcutaneous solutions, suspensions for parenteral administration; or as suppositories for rectal administration.

Suitable excipients and/or carriers include, without being limited to, diluents, binders, disintegrants, lubricants, etc. For example, the compound or a finely divided form thereof, or particles comprising the compound, are mixed with a carrier or binder substance, e.g. a mono-, di- or polysaccharide such as sugars and starch, a sugar alcohol or another polyol. For example, lactose, saccharose, sorbitol, mannitol, starch, cellulose derivatives, a binder such as polyvinylpyrrolidone, and a lubricant such as magnesium stearate, calcium stearate, polyethylene glycol, waxes, paraffin, and the like are mixed, and then compressed into tablets. The compound or a finely divided form thereof or particles containing the same may be coated by another substance. The powder mixture or particles containing the compound may also be dispensed into capsules.

The pharmaceutical composition comprising ticagrelor prepared according to the invention in a desired dose is generally suitable to treat a disease or condition of a patient in need thereof, specifically to display a desired activity against platelet aggregation, or in the treatment or prophylaxis of thrombolytic events.

Further aspects of the present invention reside in the provision of valuable intermediate compounds X to XIII useful in the synthesis of a compound of formula VIII (ticagrelor, TCG), which intermediate compounds respectively have in common the amino group protecting group Pg: 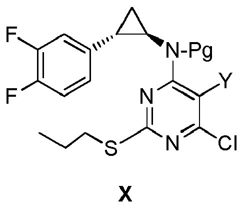 (Pg is an amino protecting group,
and Y is NO₂ or NH₂) 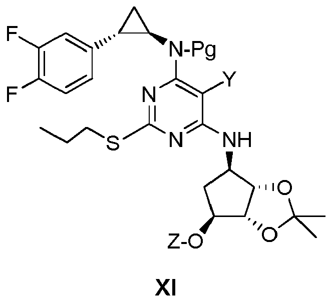 (Pg is an amino protecting group, Y is NO₂ or NH₂, and Z is hydrogen, hydroxyethyl or a group convertible to hydroxyethyl) 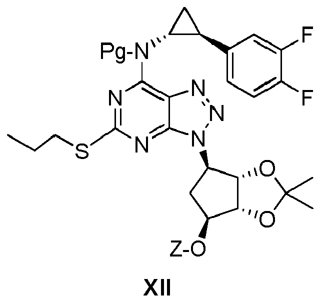 (Pg is an amino protecting group, and Z is hydrogen, hydroxyethyl or a group convertible to hydroxyethyl) 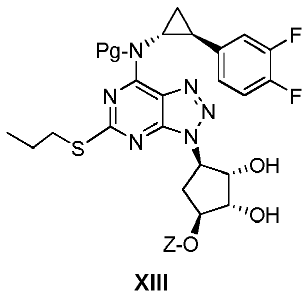 (Pg is an amino protecting group, and Z is hydrogen, hydroxyethyl or a group convertible to hydroxyethyl)

As to the definition of "Pg" and "Z", reference is made to the descriptions elsewhere in the present specification.

Particular examples of such useful intermediate compounds are listed by their respective formulas below (in these formulas, "Pr" denotes "propyl"):

| **Formula** | **Chemical name** |
|---|---|
| 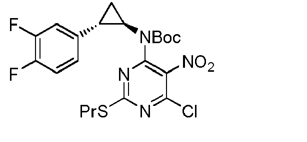 | *tert*-butyl (6-chloro-5-nitro-2-(propylthio)pyrimidin-4-yl)((1*R*,2*S*)-2-(3,4-difluorophenyl)cyclopropyl)carbamate |
| 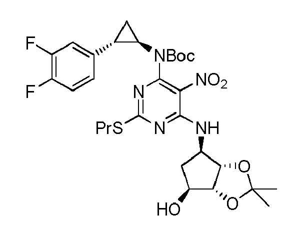 | *tert*-butyl ((1*R*,2*S*)-2-(3,4-difluorophenyl)cyclopropyl)(6-(((3a*S*,4*R*,6*S*,6a*R*)-6-hydroxy-2,2-dimethyltetrahydro-3a*H*-cyclopenta[*d*][1,3]dioxol-4-yl)amino)-5-nitro-2-(propylthio)pyrimidin-4-yl)carbamate |
| 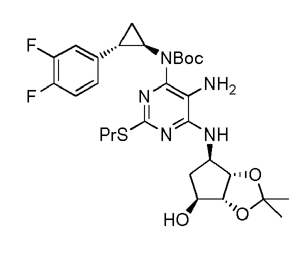 | *tert*-butyl (5-amino-6-(((3a*S*,4*R*,6*S*,6a*R*)-6-hydroxy-2,2-dimethyltetrahydro-3a*H*-cyclopenta[*d*][1,3]dioxol-4-yl)amino)-2-(propylthio)pyrimidin-4-yl)((1*R*,2*S*)-2-(3,4-difluorophenyl)cyclopropyl)carbamate |
| 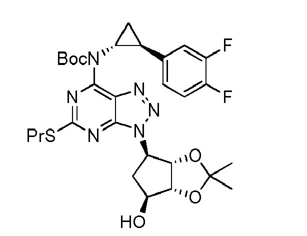 | *tert*-butyl ((1*R*,2*S*)-2-(3,4-difluorophenyl)cyclopropyl)(3-((3a*S*,4*R*,6*S*,6a*R*)-6-hydroxy-2,2-dimethyltetrahydro-3a*H*-cyclopenta[*d*][1,3]dioxol-4-yl)-5-(propylthio)-3*H*-[1,2,3]triazolo[4,5-d]pyrimidin-7-yl)carbamate |
| 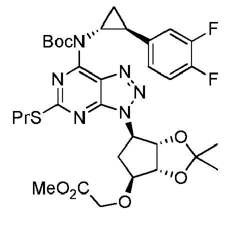 | methyl 2-(((3a*R*,4*S*,6*R*,6a*S*)-6-(7-((*tert*-butoxycarbonyl)((1*R*,2*S*)-2-(3,4-difluorophenyl)cyclopropyl)amino)-5-(propylthio)-3*H*-[1,2,3]triazolo[4,5-d]pyrimidin-3-yl)-2,2-dimethyltetrahydro-3a*H*-cyclopenta[*d*][1,3]dioxol-4-yl)oxy)acetate |
| 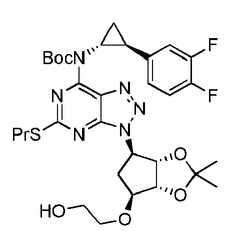 | *tert*-butyl ((1*R*,2*S*)-2-(3,4-difluorophenyl)cydopropyl)(3-((3a*S*,4*R*,6*S*,6a*R*)-6-(2-hydroxyethoxy)-2,2-dimethyltetrahydro-3a*H*-cyclopenta[*d*][1,3]dioxol-4-yl)-5-(propylthio)-3*H*-[1,2,3]triazolo[4,5-d]pyrimidin-7-yl)carbamate |

In the above particularly exemplified compounds, "Pg" is represented by a tert-butyloxycarbonyl (Boc)-group, but it should be apparent that analogous useful and further specifically exemplified intermediate compounds correspond to those listed above, wherein the specific Pg-group = Boc is replaced by other amine group protecting groups, specifically those where the tert-butyloxycarbonyl (Boc) group is replaced by carbobenzyloxy (Cbz), by benzenesulfonyl (Bs), by p-toluenesulfonyl (Ts), or by 2-naphthylsulfonyl.

Specific embodiments representing the basic synthetic concept of the present invention are further described below. More specifically, in an illustrative but non-limiting example, scheme 10 below illustrates a concept of the molecular assembly of ticagrelor (TCG), while noting that this scheme is by no means limiting with respect to the specific nature of transformation, reactions, conditions protecting groups, methods and reagents, which can be used.

This illustration of the general synthetic route outline includes the *N*-arylation of a carbamate, sulfonamide or other amide derivative of **CPA** with **CLIN.** This gives the *N*-protected intermediates of the structural type **II** which are then used to arylate **AMAL** to give intermediates **III.** The nitro group is then reduced to give the diamines **IV** which can be nitrosated to affect the formation of the triazole ring of intermediates **V.** This type of compounds is already suitably protected for the selective introduction of the hydroxyethyl group on the secondary alcohol group. The hydroxyethylation process and the later deprotection steps to give ticagrelor (**TCG**) can be performed by various methodologies known to those skilled in the art, such as an *O-*alkylation with an alkyl haloacetate, ester group reduction and deprotection under acidic conditions.

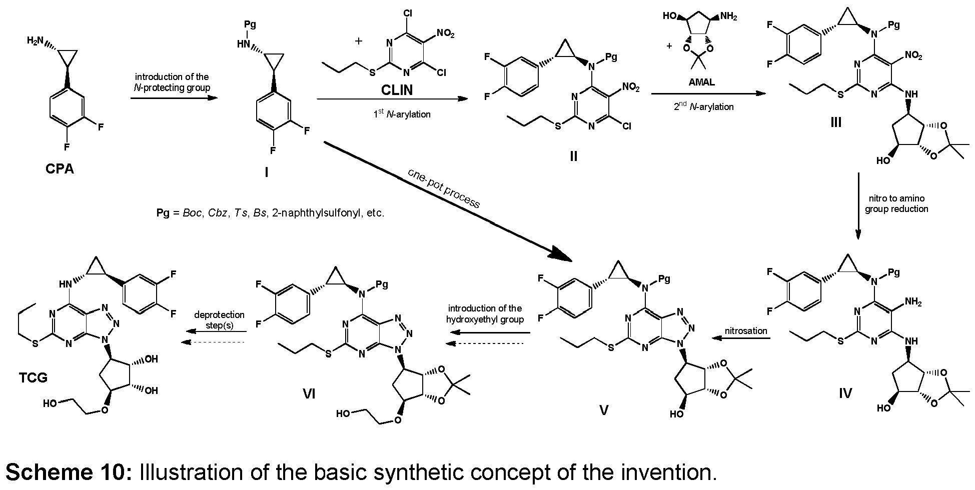

In a more specific example of ticagrelor synthesis, the embodiment of the invention is represented by the use of the *tert*-butoxycarbonyl (*Boc*) protected **CPA** derivative **CPABOC** (Scheme 10). This is deprotonated with sodium hydride in order to be *N*-arylated with **CLIN.** This gives **CPABOCCIN** with good selectivity and yields. This product is then used for the *N-*arylation of **AMAL,** preferably in the presence of a base such as triethylamine or alkali carbonates, to give **BAAL**. The next reaction steps include the reduction of the nitro group to give **BAALA** and its nitrosation to give **BATAM**. This is a key intermediate for use in the subsequent hydroxyethylation and deprotection steps which can be implemented using numerous synthetic approaches. Specifically, this can be achieved by alkylation with methyl bromoacetate to give **BATAME**, its reduction with lithium borohydride to give **BATAMA**, and finally the one step deprotection of the acetonide and Boc protecting groups under acidic conditions to give ticagrelor (**TCG**).

This synthetic route was found particularly advantageous for the aplication of one-pot methodologies. Specifically, the synthesis can be efficiently carried from **CPABOC** to **BATAM** in a one-pot procedure comprised of four chemical steps (Scheme 11).

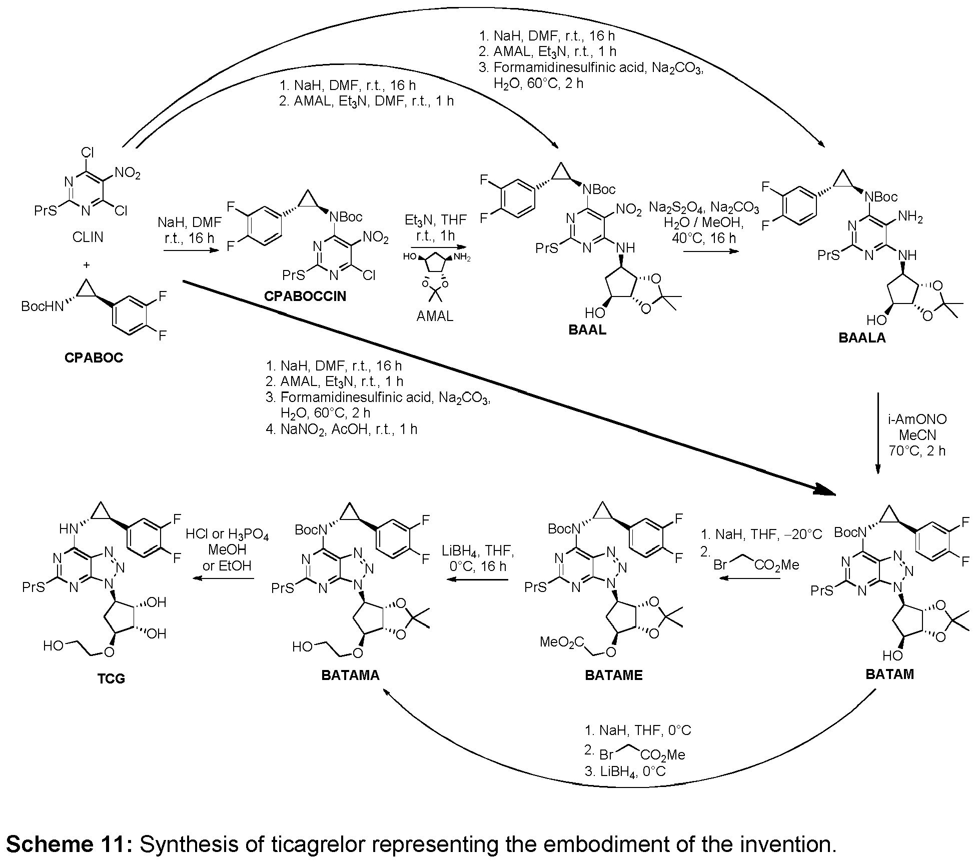

Protecting groups other than *Boc* may also be suitable. For example, the sulfonyl protecting groups, such as the p-toluenesulfonyl (*Ts*), benzenesulfonyl (*Bs*) or the 2-naphthalenesulfonyl group, were found to be suitable for the one pot synthesis of intermediates of type **V** directly from **CPA** derived sulfonamides **CPATs**, **CPAS** or **CPAN** correspondingly (Scheme 12). The increased acidity of these sulfonamides in comparison to the less acidic carbamate **CPABOC** allows for the use of more practical bases than sodium hydride. Tripotassium phosphate in acetonitrile was found particularly suitable for this first chemical step, as well as for the subsequent two chemical steps comprising the one-pot process toward **TAALA**, **SAALA** or **NAALA**. The triazole ring formation is suitably carried out by extracting the water diluted reaction mixture with ethyl acetate, phase separation, addition of isopentyl nitrite to the organic phase and mild heating. The intermediates **TAALA**, **SAALA** and **NAALA** thus need not be isolated in this process.

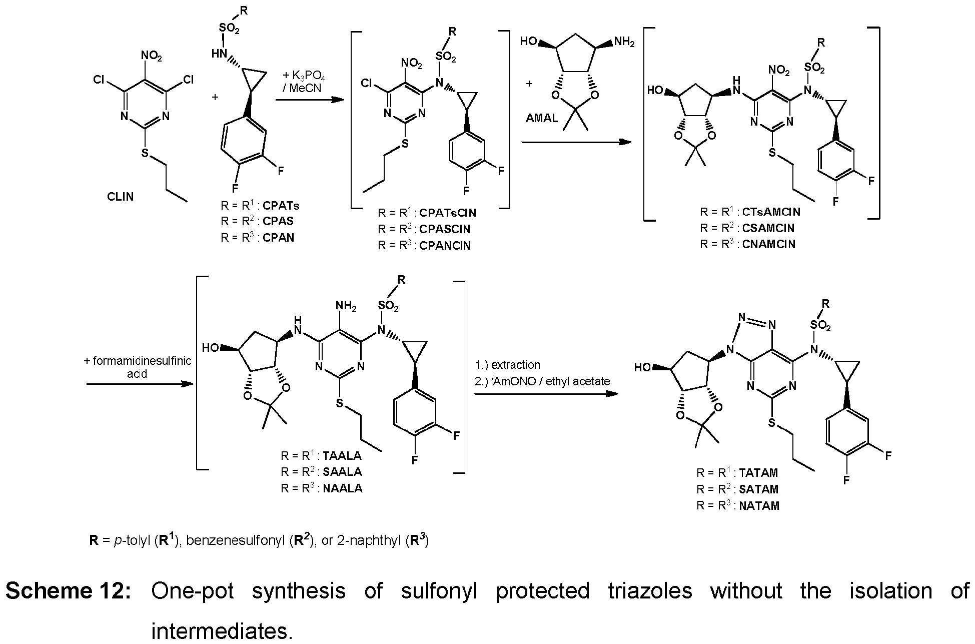

The compounds **TATAM**, **SATAM**, **NATAM**, or related sulfonamides, corresponding to intermediates of type **V**, can then be used for the introduction of the hydroxyethyl ether side chain on their hydroxy group and further transformations toward ticagrelor.

In another embodiment the key intermediate **BATAM** can be prepared from **CPATAM**, which can be derived from **CLTAM** as shown in scheme 13. The latter can be prepared as described in WO 00/34283 (Scheme 1).

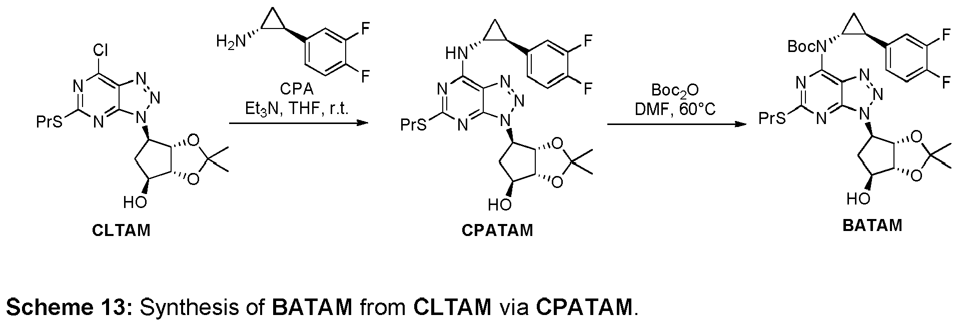

In the following the present invention will be described in further detail by illustrative, non-limiting examples.

### Examples

### Example 1: Preparation of tert-butyl ((1R,2S)-2-(3,4-difluorophenyl)cyclopropyl)carbamate (CPABOC)

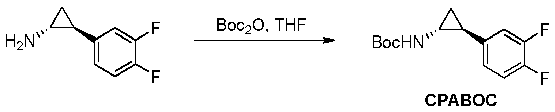

To a solution of *trans*-(1*R*,2*S*)-2-(2,3-difluorophenyl)cyclopropylamine (26.4 g, 156 mmol) (24 mL, 172 mmol) in THF (100 mL) was slowly added a solution of Boc₂O in THF (100 mL). The resulting reaction mixture was stirred at room temperature for 1 h, then concentrated. After addition of water (200 mL), the white precipitate was filtered off, washed with water (3 x 100 mL), and dried to afford 42.0 g (99% yield) of title compound as white powder. ¹H NMR (CDCl₃, 500 MHz) δ 1.07-1.16 (m, 2H), 1.45 (s, 9H), 2.01 (m, 1H), 2.31 (m, 1H), 2.64 (m, 1H), 4.91 (br s, 1H), 6.88 (m, 1H), 6.95 (m, 1H), 7.03 (m, 1H); ¹⁹F NMR (CDCl₃, 470.5 MHz) δ -139.3 (m, 1F), -142.8 (m, 1 F); MS (ESI) *m*/*z*: 270 [MH]⁺.

### Example 2: Preparation of tert-butyl (6-chloro-5-nitro-2-(propylthio)pyrimidin-4-yl)((1R,2S)-2-(3,4-difluorophenyl)cyclopropyl)carbamate (CPABOCCIN)

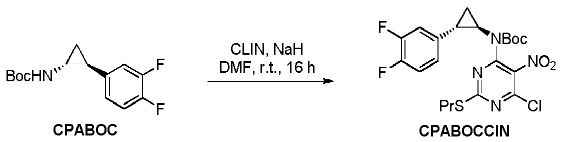

A mixture of **CPABOC** (0.20 g, 0.74 mmol), **CLIN** (0.20 g, 0.74 mmol) and NaH (60% in oil, 36 mg, 0.74 mmol) in dry DMF (2 mL) was stirred at room temperature for 16 hours, then acetic acid (0.5 mL) and water (10 mL) were added. After extraction with diisopropyl ether (3 x 10 mL), the combined organic layers were dried over MgSO₄, and concentrated. Purification by chromatography (SiO₂, hexane:EtOAc) afforded title compound as yellow oil (0.23 g, 62% yield). ¹H NMR (CDCl₃, 500 MHz) δ 0.95 (t, *J* = 7.3 Hz, 3H), 1.22 (m, 1H), 1.29 (q, *J* = 6.6 Hz, 1H), 1.36 (s, 9H), 1.66 (m, 2H), 2.17 (m, 1H), 2.94 (m, 1H), 3.03 (m, 1H), 3.08 (m, 1H), 6.58 (m, 1H), 6.94 (m, 1H), 7.01 (m, 1H).

### Example 3: Preparation of tert-butyl ((1R,2S)-2-(3,4-difluorophenyl)cyclopropyl)(6-(((3aS,4R,6S,6aR)-6-hydroxy-2,2-dimethyltetrahydro-3aH-cyclopenta[d]-[1,3]dioxol-4-yl)amino)-5-nitro-2-(propylthio)pyrimidin-4-yl)carbamate (BAAL)

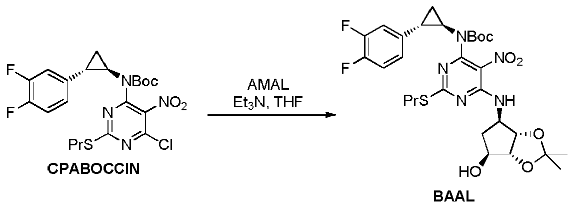

To a solution of **CPABOCCIN** (0.20 g, 0.40 mmol) and triethylamine (0.061 mL, 0.44 mmol) in dry THF (2 mL) was added **AMAL** (76 mg, 0.44 mmol). The resulting reaction mixture was stirred at room temperature for 1 h, then water was added (10 mL), and the product was extracted to THF (3 x 5 mL). Combined organic layers were dried over MgSO₄, and then concentrated. Purification by chromatography (SiO₂, hexane:EtOAc) afforded the title compound as a yellow oil (0.24 g, 93% yield). ¹H NMR (CDCl₃, 500 MHz) δ 0.96 (t, *J* = 7.3 Hz, 3H), 1.19 (s, 3H), 1.27 (m, 2H), 1.31 (s, 9H), 1.36 (s, 3H), 1.69 (m, 2H), 1.77 (m, 1 H), 2.19 (br s, 1H), 2.26 (m, 1H), 2.67 (m, 1H), 2.91-3.08 (m, 3H), 4.29 (m, 1H), 4.46 (m, 1H), 4.52 (m, 1H), 4.68 (m, 1H), 6.88 (m, 1H), 6.94-7.01 (m, 2H), 8.64 (d, *J* = 8.0 Hz, 1H); ¹⁹F NMR (CDCl₃, 470.5 MHz) δ - 139.4 (m, 1 F), -142.6 (m, 1 F); MS (ESI) *m*/*z*: 638 [MH]⁺.

**BAAL** was also prepared through one-pot reaction starting from **CPABOC.**

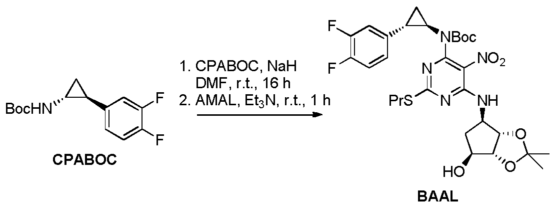

A mixture of **CPABOC** (1.0 g, 3.71 mmol), **CLIN** (1.0 g, 3.71 mmol) and NaH (60% in oil, 0.16 g, 4.08 mmol) in dry DMF (8 mL) was stirred at room temperature for 16 hours, then triethylamine (0.57 mL, 4.08 mmol) and **AMAL** (0.71 g, 4.08 mmol) were added at room temperature, and the resulting reaction mixture was stirred at room temperature for 1 h. Water (50 mL) was slowly added and product was extracted to diisopropyl ether (3 x 30 mL). Combined organic phases were dried over MgSO₄, then concentrated to afford crude compound, which was then purified by chromatography (SiO₂, hexane:EtOAc) to afford title compound as yellow oil (1.94 g, 82%).

### Example 4: Preparation of tert-butyl (5-amino-6-(((3aS,4R,6S,6aR)-6-hydroxy-2,2-dimethyltetrahydro-3aH-cyclopenta[d][1,3]dioxol-4-yl)amino)-2-(propylthio) pyrimidin-4-yl)((1R,2S)-2-(3,4-difluorophenyl)cyclopropyl)carbamate (BAALA)

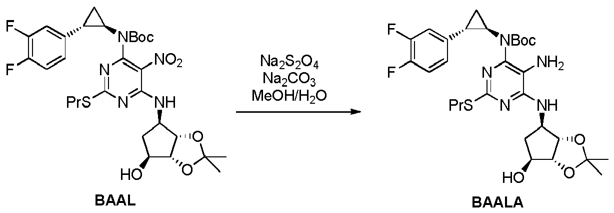

A solution of **BAAL** (0.64 g, 1.0 mmol) in MeOH (2 mL) was added to a mixture of sodium dithionite (0.57 g, 3.3 mmol), Na₂CO₃ (0.35 g, 3.3 mmol), water (1 mL) and MeOH (1 mL). Resulting reaction mixture was stirred at 40°C for 16 h, then water was added (30 mL), and product was extracted to EtOAc (3 x 20 mL). Combined organic phases were dried over MgSO₄, then concentrated to giva a crude compound, which was then purified by chromatography (SiO₂, hexane:EtOAc) to afford the title compound as an orange oil (0.41 g, 68%). ¹⁹F NMR (CDCl₃, 470.5 MHz) δ -139.4 (m, 1 F), -142.7 (m, 1 F); MS (ESI) *m*/*z*: 608 [MH]⁺.

**BAALA** was also prepared through a one-pot reaction starting from **CPABOC.**

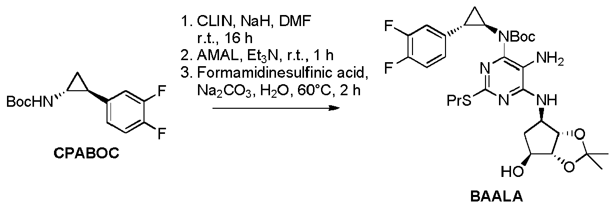

A mixture of **CPABOC** (1.0 g, 3.71 mmol), **CLIN** (1.0 g, 3.71 mmol) and NaH (60% in oil, 0.16 g, 4.08 mmol) in dry DMF (8 mL) was stirred at room temperature for 16 hours, then triethylamine (0.57 mL, 4.08 mmol) and **AMAL** (0.64 g, 3.71 mmol) were added at room temperature. The resulting reaction mixture was stirred at room temperature for 1 h. Then water (1 mL), Na₂CO₃ (1.29 g, 12.2 mmol) and formamidinesulfinic acid (1.32 g, 12.2 mmol) were added, and the resulting reaction mixture was stirred at 60°C for 2 h. Water (100 mL) was added and the product was extracted to 2-methyltetrahydrofuran (3 x 30 mL). Combined organic phases were dried over MgSO₄, then concentrated to give a crude compound, which was then purified by chromatography (SiO₂, hexane:EtOAc) to afford the title compound as a orange oil (1.42 g, 63% yield).

### Example 5: Preparation tert-butyl ((1R,2S)-2-(3,4-difluorophenyl)cyclopropyl)(3-((3aS,4R, 6S,6aR)-6-hydroxy-2,2-dimethyltetrahydro-3aH-cyclopenta[d][1,3]dioxol-4-yl)-5-(propylthio)-3H-[1,2,3]triazolo[4,5-d]pyrimidin-7-yl)carbamate (BATAM)

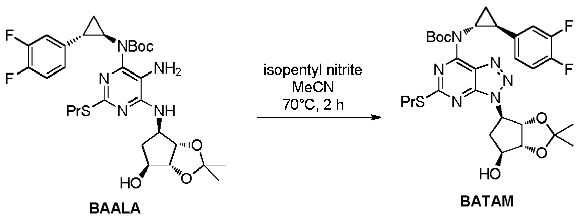

The title compound was prepared using the method described in WO 00/34283.

A solution of **BAALA** (0.40 g, 0.66 mmol) and isopentyl nitrite (0.13 mL, 1.0 mmol) in acetonitrile (4 mL) was stirred for 2 h at 70 °C. Volatile components were then evaporated, and crude product was purified by chromatography (SiO₂ hexane:EtOAc) to afford the title compound as a yellow oil (0.40 g, 98% yield). ¹H NMR (CDCl₃, 500 MHz) δ 0.99 (t, *J* = 7.3 Hz, 3H), 1.22 (m, 1H), 1.25 (s, 3H), 1.38 (m, 1H), 1.42 (s, 9H), 1.47 (s, 3H), 1.72 (m, 2H), 2.12 (m, 1H), 2.23 (m, 1H), 2.84 (m, 1H), 2.99-3.05 (m, 1H), 3.07-3.14 (m, 1H), 3.17 (m, 1H), 4.28 (m, 1H), 4.36 (m, 1H), 4.72 (m, 1H), 4.86 (m, 1H), 5.30 (m, 1H), 6.90 (m, 1H), 6.98-7.04 (m, 2H); ¹⁹F NMR (CDCl₃, 470.5 MHz) δ-139.1 (m, 1F), -142.3 (m, 1F); MS (ESI) *m*/*z*: 619 [MH]⁺.

**BATAM** was also prepared through a one-pot reaction starting from **CPABOC.**

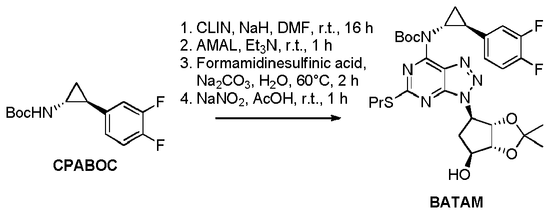

A mixture of **CPABOC** (10.0 g, 37.1 mmol), **CLIN** (9.95 g, 37.1 mmol) and NaH (60% in oil, 1.63 g, 40.8 mmol) in dry DMF (50 mL) was stirred at room temperature for 16 hours, then triethylamine (5.69 mL, 40.8 mmol), and **AMAL** (6.43 g, 37.1 mmol) were slowly added at room temperature. The resulting reaction mixture was stirred at room temperature for 1 h, then water (20 mL), Na₂CO₃ (12.9 g, 122 mmol) and formamidinesulfinic acid (13.2 g, 122 mmol) were added, and the resulting reaction mixture was stirred at 60 °C for 2 h. The reaction mixture was then cooled to 0°C. Acetic acid (50 mL) was added dropwise over 2 h. NaNO₂ (3.84 g, 55.7 mmol) was then added and resulting reaction mixture was stirred at room temperature for 1 h. Water was added (500 mL), product was extracted to 2-methyltetrahydrofuran (3 x 100 mL), combined organic phases were dried over MgSO₄, then concentrated to afford crude product, which was purified by chromatography (SiO₂, hexane:EtOAc) to afford the title compound as yellow oil (12.0 g, 52% yield).

**BATAM** was also prepared from **CLTAM** via **CPATAM.**

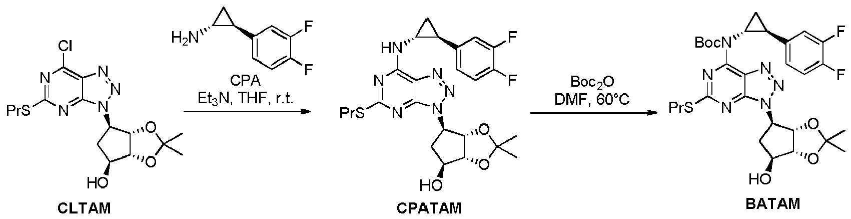

To a solution of **CLTAM,** which was prepared using the method described in WO 00/34283 (2.0 g, 5.18 mmol) and Et₃N (0.13 mL, 1.0 mmol) in dry THF (7 mL) was slowly added at room temperature a solution of CPA in dry THF (3 mL). The resulting reaction mixture was stirred for 1 h, then salts were filtered off, and the filtrate was concentrated. **CPATAM** was then crystalized from hexane/diisopropyl ether mixture to afford a white powder (2.29 g, 85% yield). Mp 108°C; ¹H NMR (CDCl₃, 500 MHz) δ 0.96 (t, J = 7.2 Hz, 3H), 1.31 (s, 3H), 1.35-1.44 (m, 2H), 1.53 (s, 3H), 1.67 (m, 2H), 1.92 (m, 1H), 2.15 (m, 1H), 2.23 (m, 1H), 2.92 (m, 1H), 3.00 (m, 1H), 3.09 (m, 1H), 3.15 (m, 1H), 4.44 (m, 1H), 4.83 (m, 1H), 4.89 (m, 1H), 5.35 (d, J = 8.4 Hz, 1H), 5.46 (m, 1H), 7.00 (m, 1H), 7.07-7.13 (m, 2H); ¹⁹F NMR (CDCl₃, 470.5 MHz) δ -139.0 (m, 1 F), -142.3 (m, 1 F); MS (ESI) m/z: 519 [MH]⁺.

A solution of **CPATAM** (2.0 g, 3.86 mmol) and Boc₂O (0.93 g, 4.26 mmol) in DMF (10 mL) was stirred at 60 °C until TLC and HPLC analysis showed total conversion (several days). Then water (100 mL) was added, and product was extracted to 2-methyltetrahydrofuran (3 x 20 mL). The combined organic phases were dried over MgSO₄, and concentrated to give a crude product, which was then purified by chromatography (SiO₂, hexane:EtOAc) to afford a yelowish oil (0.82 g, 34% yield ¹H NMR (CDCl₃, 500 MHz) δ 0.99 (t, *J* = 7.3 Hz, 3H), 1.22 (m, 1H), 1.25 (s, 3H), 1.38 (m, 1H), 1.42 (s, 9H), 1.47 (s, 3H), 1.72 (m, 2H), 2.12 (m, 1H), 2.23 (m, 1H), 2.84 (m, 1H), 2.99-3.05 (m, 1H), 3.07-3.14 (m, 1H), 3.17 (m, 1H), 4.28 (m, 1H), 4.36 (m, 1H), 4.72 (m, 1H), 4.86 (m, 1H), 5.30 (m, 1H), 6.90 (m, 1H), 6.98-7.04 (m, 2H); ¹⁹F NMR (CDCl₃, 470.5 MHz) δ -139.1 (m, 1 F), -142.3 (m, 1 F); MS (ESI) *m*/*z*: 619 [MH]⁺.

### Example 6: Preparation of methyl 2-(((3aR,4S,6R,6aS)-6-(7-((tert-butoxycarbonyl)((1R,2S)-2-(3,4-difluorophenyl)cyclopropyl)amino)-5-(propylthio)-3H-[1,2,3]triazolo[4,5-d] pyrimidin-3-yl)-2,2-dimethyltetrahydro-3aH-cyclopenta[d][1,3]dioxol-4-yl)oxy) acetate (BATAME)

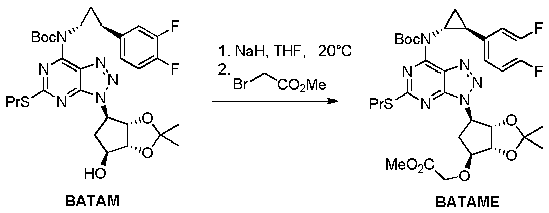

To a solution of **BATAM** (1.79 g, 2.89 mmol) in dry THF (10 mL) NaH (0.13 g, 3.18 mmol) was added at -20°C and stirred for 1 h, then methyl bromoacetate (0.30 mL, 3.18 mmol) was added at -20°C. The resulting reaction mixture was stirred at -20°C for 16 h. Acetic acid (5 mL) was added slowly, then water (50 mL) was added, and the product was extracted to 2-methyltetrahydrofuran (3 x 100 mL). The combined organic phases were washed with saturated NaHC0₃ (3 x 10 mL), dried over MgSO₄, and concentrated to give a crude product, which was then purified by chromatography (SiO₂, hexane:EtOAc). Colorless oil (1.60 g, 80% yield). ¹H NMR (CDCl₃, 500 MHz) δ 0.99 (t, *J* = 7.3 Hz, 3H), 1.21 (m, 1H), 1.28 (s, 3H), 1.36 (m, 1H), 1.40 (s, 9H), 1.48 (s, 3H), 1.73 (m, 2H), 2.14 (m, 1H), 2.68 (m, 2H), 3.00-3.11 (m, 2H), 3.18 (m, 1H), 3.65 (s, 3H), 4.01-4.10 (m, 3H), 4.76 (dd, *J* = 6.8, 2.5 Hz, 1H), 5.11 (m, 1H), 5.42 (dd, *J* = 6.8, 3.7 Hz, 1H), 6.91 (m, 1H), 6.97-7.05 (m, 2H); ¹⁹F NMR (CDCl₃, 470.5 MHz) δ -139.4 (m, 1F), - 142.5 (m, 1 F); MS (ESI) *m*/*z*: 691 [MH]⁺.

### Example 7: Preparation of tert-butyl ((1R,2S)-2-(3,4-difluorophenyl)cyclopropyl)(3-((3aS,4R, 6S,6aR)-6-(2-hydroxyethoxy)-2,2-dimethyltetrahydro-3aH-cyclopenta[d][1,3] dioxol-4-yl)-5-(propylthio)-3H-[1,2,3]triazolo[4,5-d]pyrimidin-7-yl)carbamate (BATAMA)

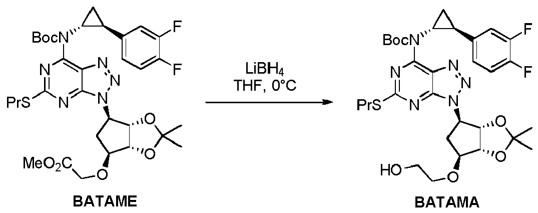

To a solution of **BATAME** (1.79 g, 2.89 mmol) in dry THF (10 mL) at 0°C LiBH₄ (0.10 g, 4.63 mmol) was added and stirred for 16 h. The reaction was quenched by addition of saturated NaHCO₃ solution (5 mL). Water was added (50 mL), product was extracted to MeTHF (3 x 30 mL), combined organic phases were dried over MgSO₄, then concentrated to give a crude product, which was purified by chromatography (SiO₂, hexane:EtOAc) to afford the title compound as a colorless oil (1.20 g, 78% yield). ¹H NMR (CDCl₃, 500 MHz) δ 1.07 (t, *J* = 7.3 Hz, 3H), 1.29 (m, 1H), 1.37 (s, 3H), 1.43 (q, *J* = 6.9 Hz, 1H), 1.48 (s, 9H), 1.56 (s, 3H), 1.80 (m, 2H), 2.21 (m, 1H), 2.26 (m, 1H), 2.56 (m, 1H), 2.70 (m, 1H), 3.07-3.20 (m, 2H), 3.26 (m, 1H), 3.49 (m, 1H), 3.52-3.66 (m, 3H), 4.05 (m, 1H), 4.87 (d, *J* = 6.4 Hz, 1H), 5.22 (m, 1H), 5.53 (m, 1H), 6.98 (m, 1H), 7.04-7.12 (m, 2H); ¹⁹F NMR (CDCl₃, 470.5 MHz) δ -139.3 (m, 1 F), -142.4 (m, 1 F); MS (ESI) *m*/*z*: 663 [MH]⁺.

**BATAMA** was also prepared through a one-pot reaction starting from **BATAM.**

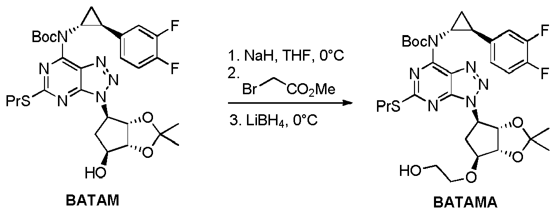

To a solution of **BATAM** (2.0 g, 3.23 mmol) in dry THF (10 mL) NaH (60% in oil, 0.14 g, 3.56 mmol) was added at 0 °C and stirred for 15 min, then methyl bromoacetate (0.36 mL, 3.80 mmol) was added, and the resulting reaction mixture was stirred at 0 °C for 2 h. Then LiBH₄ (0.14 g, 6.46 mmol) was added at 0°C and stirred for 2 h. The reaction was quenched by addition of saturated NaHCO₃ solution (5 mL). Water was added (20 mL), product was extracted to MeTHF (3 x 10 mL), combined organic phases were dried over MgSO₄, then concentrated to give a crude product, which was purified by chromatography (SiO₂, hexane:EtOAc) to afford the title compound as colorless oil (0.42 g, 20% yield).

### Example 8: Preparation of (1S,2S,3R,5S)-3-(7-(((1R,2S)-2-(3,4-difluorophenyl)cyclopropyl) amino)-5-(propylthio)-3H-[1,2,3]triazolo[4,5-d]pyrimidin-3-yl)-5-(2-hydroxyethoxy) cyclopentane-1,2-diol (TCG, ticagrelor)

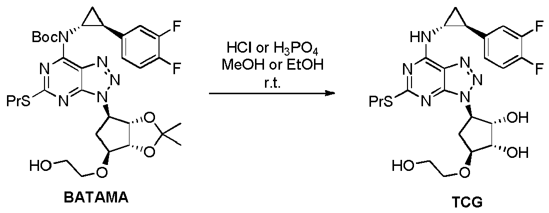

To a solution of **BATAMA** (0.25 g, 0.38 mmol) in EtOH (10 mL) at room temperature *ortho*phosphoric acid (85%, 1.5 mL) was slowly added. The resulting reaction mixture was stirred at room temperature for 24 h. Water was then added (20 mL) and the reaction mixture neutralized with 1M NaOH. The product was extracted to ethyl acetate (5 x 10 mL), the combined organic phases were dried over Na₂SO₄, then concentrated to give a crude product, which was purified by chromatography (SiO₂, EtOAc) to afford title compound as a white powder (0.17 g, 87% yield). ¹⁹F NMR (CD₃OD, 470.5 MHz) δ -141.9 - -142.1 (m, 1F), -145.6 - -145.9 (m, 1F); MS (ESI) *m*/*z*: 523 [MH]⁺.

### Example 9: Preparation of N-((1R,2S)-2-(3,4-difluorophenyl)cyclopropyl)-4-methylbenzen esulfonamide (CPATs)

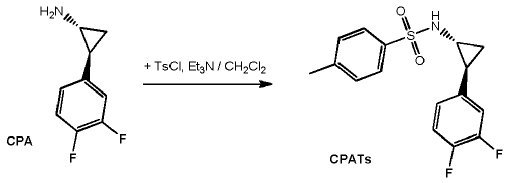

To a solution of **CPA** (16.92 g, 100 mmol) and triethylamine (17.4 mL, 125 mmol) in dichloromethane (140 mL) stirring in an ice bath was drop-wise added a solution of *p-*toluenesulfonyl chloride (20.02 g, 105 mmol) in dichloromethane (50 mL) in the course of 30 min. After 1 h reaction time there was added NH₃(aq) (25%, 10 mL), the mixture was left stirring for additional 10 min and then washed with water (300 mL), 1M HCl(aq) (150 mL), water (300 mL) and evaporated under reduced pressure to give a white solid (31.45 g, 97%): ¹H NMR (CDCl₃, 500 MHz) δ 1.06 (q, *J* = 6.8 Hz, 1H), 1.26 (m, 1H), 2.11 (m, 1H), 2.31 (m, 1H), 2.43 (s, 3H), 5.33 (s, 1H), 6.74 (m, 2H), 7.02 (m, 1H), 7.29 (d, *J* = 8.0 Hz, 2H), 7.73 (d, *J* = 8.0 Hz, 2H); ¹⁹F NMR (CDCl₃, 470.5 MHz) δ -138.89 (m, 1 F), -142.17 (m, 1 F).

### Example 10: Preparation of N-((1R,2S)-2-(3,4-difluorophenyl)cyclopropyl)naphthalene-2-sulfonamide (CPAN)

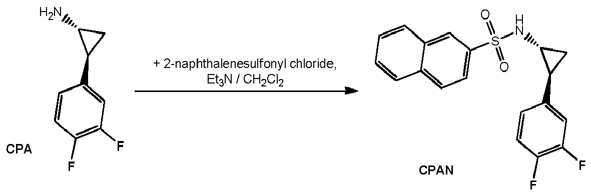

Prepared in the same manner as **CPATs** (Example 9) by using 2-naphthalenesulfonyl chloride (11.90 g, 52.5 mmol) giving **CPAN** as a white solid (17.43 g, 97%): ¹⁹F NMR (CDCl₃, 470.5 MHz) δ -138.78 (m, 1 F), -142.11 (m, 1 F).

### Example 11: Preparation of N-(6-chloro-5-nitro-2-(propylthio)pyrimidin-4-yl)-N-((1R,2S)-2-(3,4-difluorophenyl)cyclopropyl)-4-methylbenzenesulfonamide (CPTsCIN)

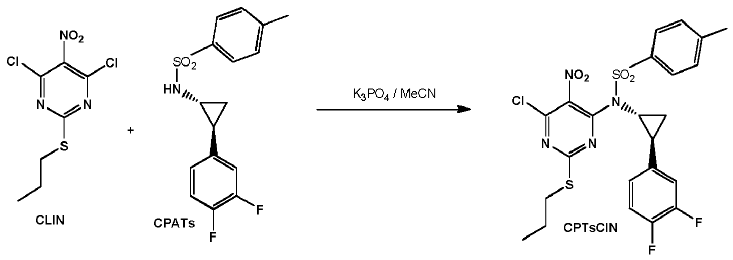

To a solution of **CPATs** (1.29 g, 4 mmol) and **CLIN** (1.07 g, 4 mmol) in acetonitrile (15 mL) was added anhydrous K₃PO₄ (1.27 g, 6 mmol) and the mixture stirred for 24 h at 25 °C. The reaction mixture was then diluted with water (50 mL), extracted with diisopropyl ether (50 mL), the extract washed with water (2 x 50 mL) and evaporated under reduced pressure. The crude product was purified with flash chromatography to give a brownish resin (1.08 g, 49%): MS (ESI) *m*/*z*: 555 [MH]⁺; ¹⁹F NMR (CDCl₃, 470.5 MHz) δ -138.43 (m, 1 F), -141.32 (m, 1 F).

### Example 12: Preparation of N-(6-chloro-5-nitro-2-(propylthio)pyrimidin-4-yl)-N-((1R,2S)-2-(3,4-difluorophenyl)cyclopropyl)naphthalene-2-sulfonamide (CPNCIN)

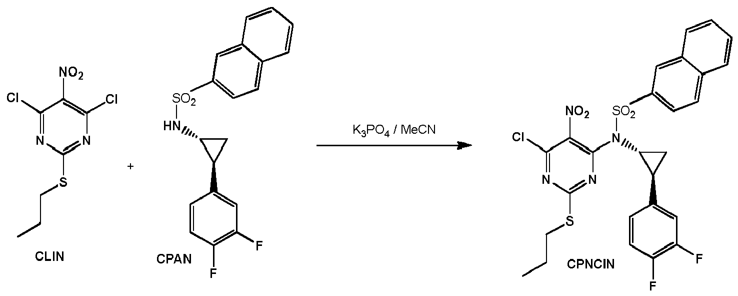

To a solution of **CPAN** (1.44 g, 4 mmol) and **CLIN** (1.07 g, 4 mmol) in acetonitrile (15 mL) was added anhydrous K₃PO₄ (1.27 g, 6 mmol) and the mixture stirred for 48 h at 25 °C. The reaction mixture was then diluted with water (50 mL), extracted with diisopropyl ether (50 mL), the extract washed with water (2 x 50 mL) and evaporated under reduced pressure. The crude product was purified with flash chromatography to give a brownish resin (1.56 g, 67%): MS (ESI) m/z: 591 [MH]⁺.

### Example 13: Preparation of N-((1R,2S)-2-(3,4-difluorophenyl)cyclopropyl)-N-(6-(((3aS,4R,6S, 6aR)-6-hydroxy-2,2-dimethyltetrahydro-3aH-cyclopenta[d][1,3]dioxol-4-yl)amino)-5-nitro-2-(propylthio)pyrimidin-4-yl)-4-methylbenzenesulfonamide (CTsAMCIN)

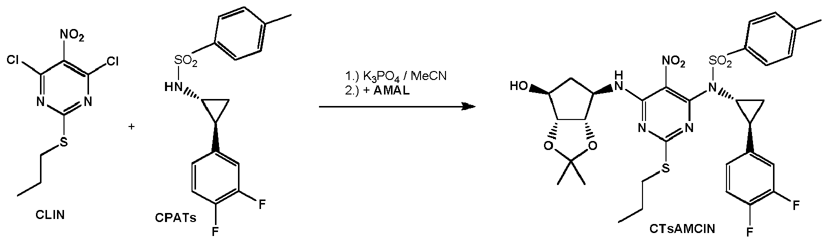

To a solution of **CPATs** (1.29 g, 4 mmol) and **CLIN** (1.07 g, 4 mmol) in acetonitrile (15 mL) was added anhydrous K₃PO₄ (1.27 g, 6 mmol) and the mixture stirred for 24 h at 25 °C. **AMAL** (0.66 g, 3.8 mmol) was then added and stirring continued for additional 2 h. The reaction mixture was then diluted with water (50 mL), extracted with diisopropyl ether (50 mL), the extract washed with water (2 × 50 mL) and evaporated under reduced pressure. The crude product was purified with flash chromatography to give an amorphous solid (2.14 g, 77%): 95 area% HPLC; MS (ESI) *m*/*z*: 692 [MH]⁺; ¹⁹F NMR (CDCl₃, 470.5 MHz) δ -138.93 (m, 1 F), -141.97 (m, 1 F).

### Example 14: Preparation of N-(5-amino-6-(((3aS,4R,6S,6aR)-6-hydroxy-2,2-dimethyltetrahydro -3aH-cyclopenta[d][1,3]dioxol-4-yl)amino)-2-(propylthio)pyrimidin-4-yl)-N-((1R,2S) -2-(3,4-difluorophenyl)cyclopropyl)-4-methylbenzenesulfonamide (TAALA)

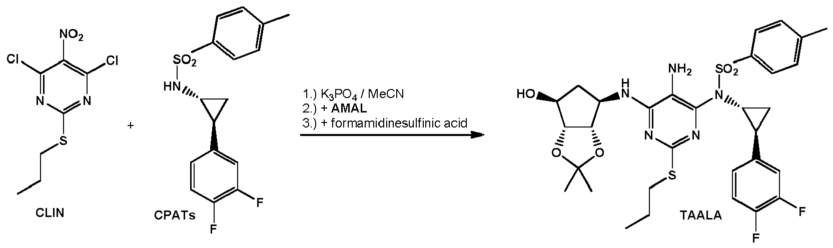

A mixture of **CLIN** (0.96 g, 3.6 mmol), **CPATs** (1.29 g, 4 mmol) and K₃PO₄ (2.29 g, 10.8 mmol) in acetonitrile (15 mL) was stirred for 120 min at 25 °C. **AMAL** (0.66 g, 3.8 mmol) was added and the mixture stirred for additional 2 hours. At this point formamidinesulfinic acid (1.36 g, 12.6 mmol) was added and the reaction temperature increased to 60 °C. After 24 hours, the mixture was diluted with water (75 mL), extracted with with ethyl acetate (50 mL), the extract washed with 0.1M HCl(aq) (50 mL) and evaporated on a rotavapor to give a pale orange amorphous solid which was further purified by flash chromatography with hexane / ethyl acetate eluent (gradient from 4 : 1 to 1 : 1) to give **TAALA** as an light brown amorphous powder (1.20 g, 50%): MS (ESI) *m*/*z*: 662 [MH]⁺.

### Example 15: Preparation of N-((1R,2S)-2-(3,4-difluorophenyl)cyclopropyl)-N-(3-((3aS,4R,6S, 6aR)-6-hydroxy-2,2-dimethyltetrahydro-3aH-cyclopenta[d][1,3]dioxol-4-yl)-5-(propylthio)-3H-[1,2,3]triazolo[4,5-d]pyrimidin-7-yl)-4-methylbenzenesulfonamide (TATAM)

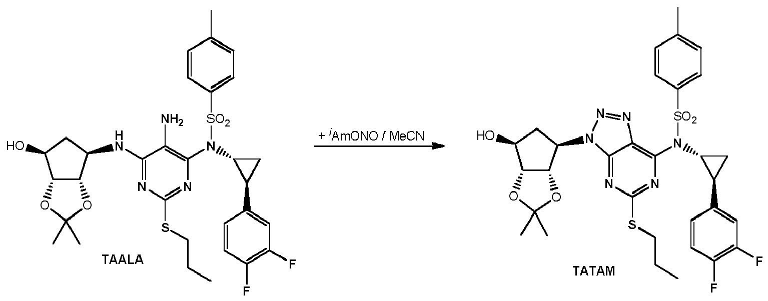

To a solution of **TAALA** (500 mg, 0.75 mmol) in acetonitrile (5 mL) was added isopentyl nitrite (0.13 mL, 0.94 mmol) and the mixture stirred for 2 h at 60 °C. The reaction mixture was then diluted with water (50 mL), extracted with diisopropyl ether (30 mL), the extract washed with water (2 × 50 mL) and evaporated under reduced pressure to give an off-white amorphous powder (450 mg, 89%): 97 area% HPLC; MS (ESI) *m*/*z*: 673 [MH]⁺.

**TATAM** was also prepared from **CLTAM** via **CPATs.**

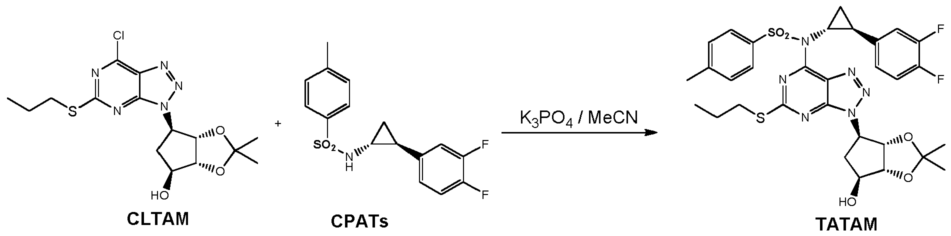

To a solution of **CLTAM** (1.16 g, 3 mmol), which was prepared using the method described in WO 00/34283, and **CPATs** (0.97 g, 3 mmol) in acetonitrile (20 mL) was added anhydrous K₃PO₄ (0.96 g, 4.5 mmol). After stirring for 24 h at 25 °C, the mixture was diluted with water (80 mL) and extracted with diisopropyl ether (50 mL). The extract was washed with water (80 mL) and evaporated under reduced pressure to give a resinous crude product which was purified by flash chromatography to afford **TATAM** as an amorphous powder (1.52 g, 75%).

### Example 16: Preparation of N-((1R,2S)-2-(3,4-difluorophenyl)cyclopropyl)-N-(3-((3aS,4R,6S, 6aR)-6-hydroxy-2,2-dimethyltetrahydro-3aH-cyclopenta[d][1,3]dioxol-4-yl)-5-(propylthio)-3H-[1,2,3]triazolo[4,5-d]pyrimidin-7-yl)naphthalene-2-sulfonamide (NATAM)

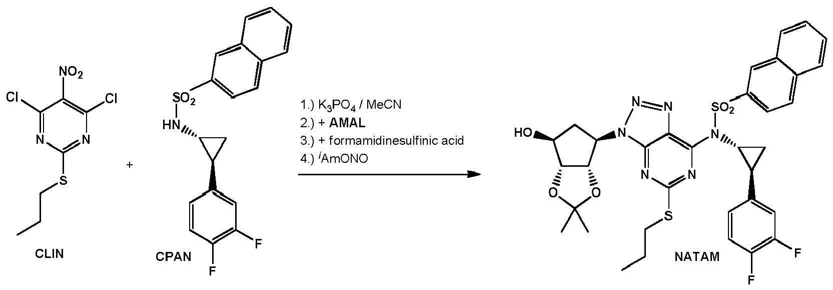

A mixture of **CLIN** (1.27 g, 4.75 mmol), **CPAN** (1.80 g, 5 mmol) and K₃PO₄ (3.03 g, 14.25 mmol) in acetonitrile (20 mL) was stirred for 150 min at 25 °C. **AMAL** (0.78 g, 4.5 mmol) was added and the mixture stirred for additional 2 hours. At this point formamidinesulfinic acid (2.05 g, 19 mmol) was added and the reaction temperature increased to 60 °C. After 18 hours, the mixture was diluted with water (100 mL), extracted with ethyl acetate (60 mL), the extract washed with 0.1 M HCl(aq) (60 mL) and evaporated under reduced pressure to give a brown resin (3.18 g). This was dissolved in ethyl acetate (30 mL), isopentyl nitrite (0.76 mL, 5.63 mmol) was added and the solution stirred for 1 h at 60 °C. The brown solution was concentrated and the product isolated using flash chromatography with hexane / ethyl acetate eluent (gradient from 5 : 1 to 2 : 1). **NATAM** was thus obtained as a beige powder (1.86 g, 58%).

### Example 17: Preparation of benzyl ((1R,2S)-2-(3,4-difluorophenyl)cyclopropyl)carbamate (CPAZ)

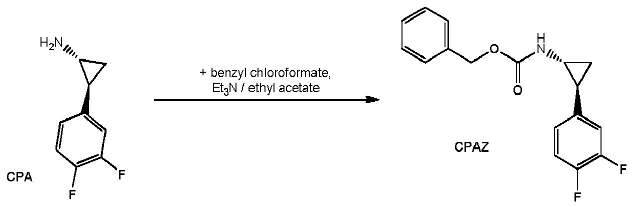

To a solution of **CPA** (11.84 g, 70 mmol) and triethylamine (12.2 mL, 87.5 mmol) in ethyl acetate (80 mL) stirring in an ice bath was drop-wise added a solution of benzyl chloroformate (11.0 mL, 77 mmol) in ethyl acetate (40 mL) at such a rate to maintain the reaction temperature bellow 15 °C. After 1 h stirring in the ice bath, water (120 mL) was added and the mixture kept stirring for additional 30 min. The organic phase was separated, washed with 0.2M HCl(aq) (200 mL), water (2×100 mL) and evaporated on a rotavapor. There was obtained a white solid (22.28 g) which was recrystallized from cyclohexane to give the product as a white crystaline solid (19.62 g, 92%): ¹H NMR (CDCl₃, 500 MHz) δ 1.17 (m, 2H), 2.07 (m, 1H), 2.71 (m, 1H), 5.15 (s, 2H), 5.35 (bs, 1H), 6.70-7.10 (m, 3H), 7.33-7.42 (m, 5H); ¹⁹F NMR (CDCl₃, 470.5 MHz) δ - 139.10 (m, 1F), -142.53 (m, 1F).

### Example 18: Preparation of benzyl (6-chloro-5-nitro-2-(propylthio)pyrimidin-4-yl)((1R,2S)-2-(3,4-difluorophenyl)cyclopropyl)carbamate (CPZCIN)

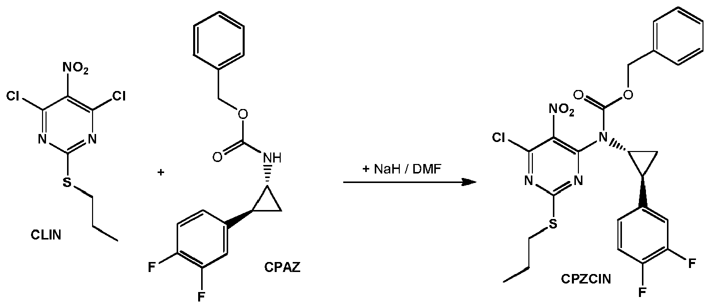

To a solution of **CPAZ** (4.85 g, 16 mmol) and **CLIN** (5.35 g, 20 mmol) in DMF (75 mL) stirring under a nitrogen atmosphere on an ice-bath was added 60% sodium hydride in oil (0.80 g, 20 mmol). After 1 h in an ice-bath, the reaction is stirred for 19 h at 25 °C. The mixture is then diluted with 1% aqueous acetic acid (300 mL), extracted with ethyl acetate (200 mL), the extract washed with water (3 × 300 mL) and evaporated under reduced pressure. The crude product was purified by flash chromatography to give a yellow resin (5.31 g, 62%).

### Example 19: Preparation of 6-chloro-N-((1R,2S)-2-(3,4-difluorophenyl)cyclopropyl)-5-nitro-2-(propylthio)pyrimidin-4-amine (CPACIN)

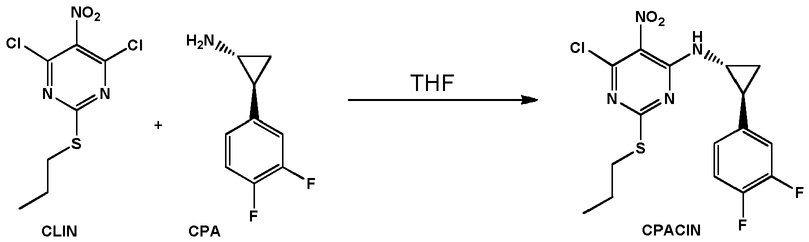

To a solution of **CLIN** (13.4 g, 50 mmol) in **THF** (150 mL) was added a solution of **CPA** (8.50 g, 50 mmol) at an addition rate of 10 mL/h while maintaining the reaction temperature at 0 °C. After the addition, the reaction mixture was then stirred at 25 °C for 4 days. *n-*Hexane (200 mL) was then added and the mixture diluted with water (500 mL). The organic phase was then washed with water and evaporated under reduced pressure to give a crude product which was purified by flash chromatography to afford **CPACIN** as a crystalline product (12.2 g, 61 %): MS (ESI) *m*/*z*: 401 [MH]⁺.

## Claims

1. Process for the preparation of a compound of formula Va 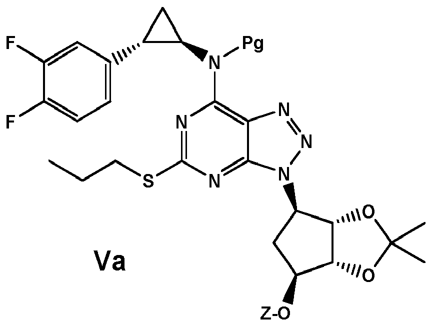 wherein Pg is an amino protecting group, and Z is hydrogen, hydroxyethyl or a group convertible to hydroxyethyl,
the process comprising the steps of:
(i) reacting a compound of formula II 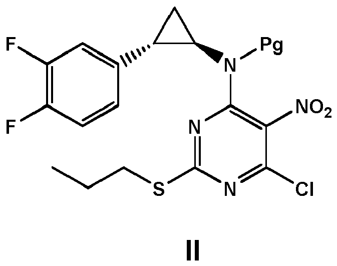 wherein Pg is defined as above, with a compound of the formula VIIa 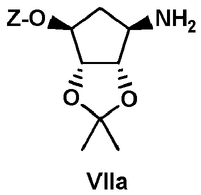 wherein Z is defined as above, to obtain a compound of formula IIIa 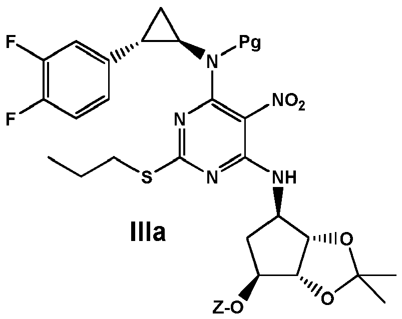 wherein Pg and Z are as defined above,
(ii) reducing the nitro group in the compound of formula IIIa to an amino group to obtain a compound of formula IVa, 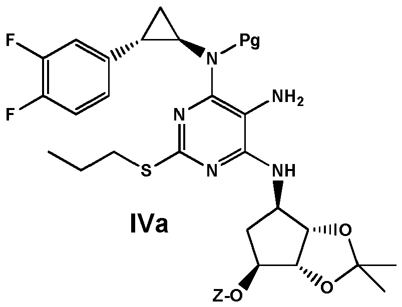 and
(iii) nitrosation of the compound of formula IVa to obtain the compound of formula Va.

2. The process according to claim 1, wherein the compound of formula II is prepared by comprising the steps of
either
(0-1) providing a compound of formula I 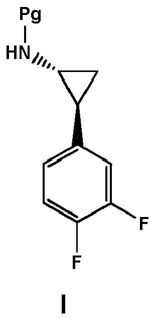 wherein Pg is an amino protecting group, and
(0-2) reacting the compound of formula I with a compound of the formula 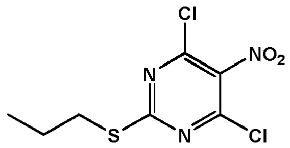 to obtain the compound of formula II;
or
(0-1') providing a compound of formula la 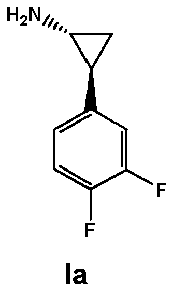
(0-2') reacting the compound of formula la with a compound of the formula 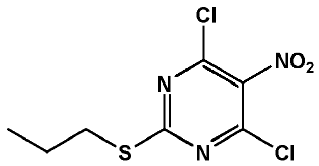 to obtain a compound of formula IIa, 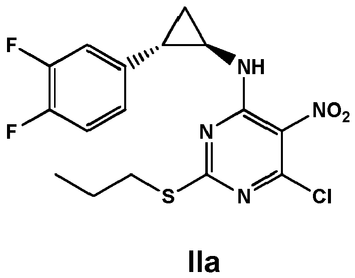 and
(0-3') introducing an amino protecting group Pg to obtain the compound of formula II.

3. The process according to claim 1 or 2, wherein the steps (i) to (iii), optionally also the preparation of the compound of formula II, are carried out in one pot.

4. The process according to any one of the preceding claims, wherein steps (i) and (ii), optionally also steps (0-1) to (0-2) or (0-1') to (0-3'), are carried out under basic conditions in the presence of bases, and/or step (iii) is carried out under acidic conditions.

5. The process according to any one of the preceding claims, wherein Z is hydrogen or a group convertible to hydroxyethyl, and after the preparation of the compound of formula Va, a hydroxyethyl group is introduced to obtain a compound of formula VI

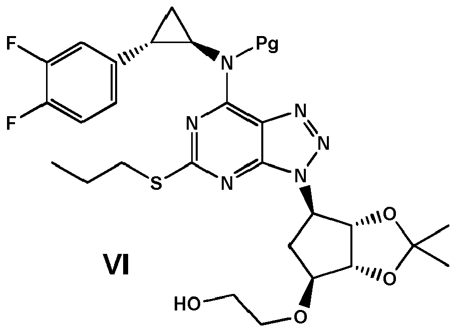

6. The process according to any one of the preceding claims, wherein Pg is selected from the group of oxycarbonyl-type amino protecting groups, carbamate-type amino protecting groups and sulfonate-type amino protecting groups, preferably Pg is selected from the group consisting of tert-butyloxycarbonyl (Boc), carbobenzyloxy (Cbz), benzenesulfonyl (Bs), p-toluenesulfonyl (Ts), and 2-naphthylsulfonyl.

7. Process for the preparation of a compound of formula VIII or a salt thereof 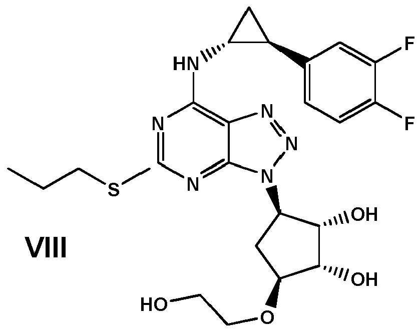 comprising the steps of:
(i) preparing a compound of formula Va or VI according to any one of claims 1 to 6 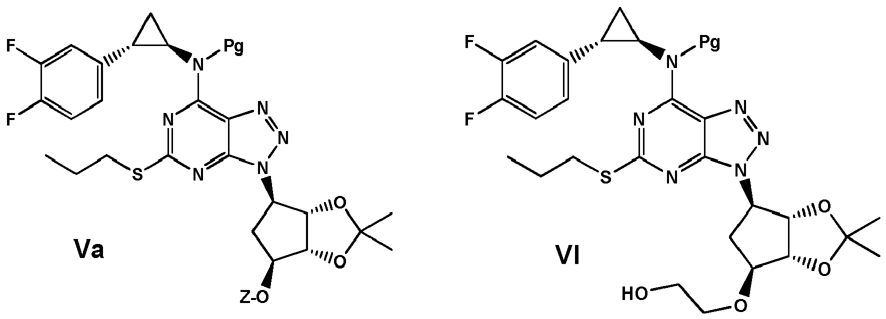 wherein Pg and Z are as defined above,
wherein if Z in the compound of formula Va is hydrogen or a group convertible to hydroxyethyl, a hydroxyethyl group is introduced to obtain a compound of formula VI,
(ii) carrying out deprotection reaction(s) to remove Pg and the vicinal hydroxyl protecting group at the pentane ring, respectively,
(iii) optionally forming a salt of the compound of formula VIII.

8. Process for the preparation of a pharmaceutical composition comprising a compound of
formula VIII or a salt thereof 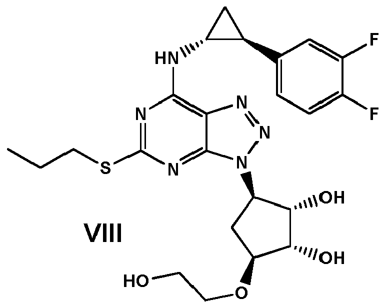 comprising the steps of:
(i) preparing a compound of formula VIII or a salt thereof according to claim 7, and
(ii) mixing the compound of formula VIII or a salt thereof with a pharmaceutically acceptable carrier and/or excipient.

9. A compound of the following formula X 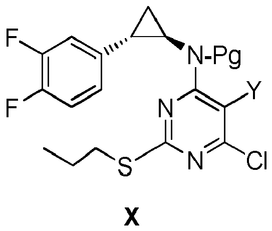 wherein Pg is an amino protecting group, and Y is NO₂ or NH₂.

10. A compound of the following formula XI 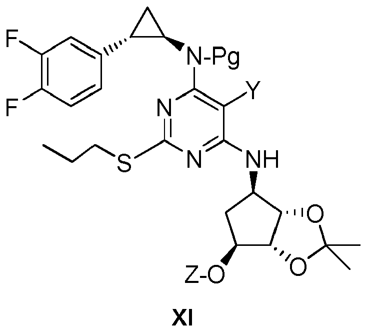 wherein Pg is an amino protecting group, Y is NO₂ or NH₂, and Z is hydrogen, hydroxyethyl or a group convertible to hydroxyethyl.

11. A compound of the following formula XII 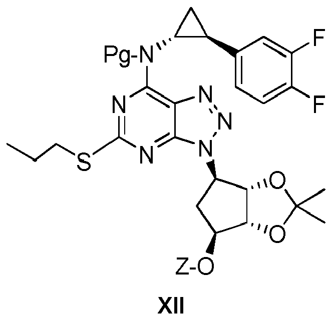 wherein Pg is an amino protecting group, and Z is hydrogen, hydroxyethyl or a group convertible to hydroxyethyl.

12. A compound of the following formula XIII 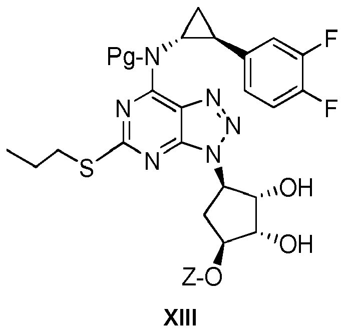 wherein Pg is an amino protecting group, and Z is hydrogen, hydroxyethyl or a group convertible to hydroxyethyl.

13. A compound as defined in any one of claims 9 to 12, wherein Pg is selected from the group of oxycarbonyl-type amino protecting groups, carbamate-type amino protecting groups and sulfonate-type amino protecting groups, preferably Pg is selected from the group consisting of tert-butyloxycarbonyl (Boc), carbobenzyloxy (Cbz), benzenesulfonyl (Bs), p-toluenesulfonyl (Ts), and 2-naphthylsulfonyl.

14. A compound as defined in any one of claims 10 to 13, wherein Z is selected from hydrogen, hydroxyethyl and (CH₃)O₂C-(CH₂)-.

15. A use of a compound as defined in any one of claims 9 to 14 in the preparation of ticagrelor.
